(19) Europäisches Patentamt
European Patent Office
Office européen des brevets

(11) Publication number: **0 178 946**
**A2**

# (12) EUROPEAN PATENT APPLICATION

(21) Application number: 85307553.9

(22) Date of filing: 18.10.85

(51) Int. Cl.[4]: **C 07 D 207/09**
C 07 D 211/34, C 07 D 223/04
C 07 D 401/06, A 61 K 31/40
A 61 K 31/44, A 61 K 31/445
A 61 K 31/495, A 61 K 31/535
A 61 K 31/55
//C07D207/416, C07D207/12,
C07D211/96, C07D211/46,
C07D223/08

(30) Priority: 19.10.84 US 662584

(43) Date of publication of application:
23.04.86 Bulletin 86/17

(84) Designated Contracting States:
AT BE CH DE FR GB IT LI LU NL SE

(71) Applicant: A.H. ROBINS COMPANY, INCORPORATED
1407 Cummings Drive P.O. Box 26609
Richmond Virginia 23261(US)

(72) Inventor: Welstead, William John, Jr.
8306 Brookfield Road
Richmond Virginia 23227(US)

(74) Representative: Sheard, Andrew Gregory et al,
Kilburn & Strode 30, John Street
London WC1N 2DD(GB)

(54) **1-(Aminoalkyl)-alpha, alpha-diaryl pyrrolidine-piperidine- and homopiperidine- acetamides and acetonitriles.**

(57) 1-(Aminoalkyl)-α, α-diaryl pyrrolidinyl, piperidino and homopiperidinoacetamides and acetonitriles having the formula

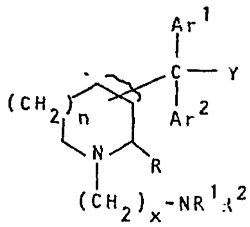

wherein:

n is 0, 1 or 2;

$Ar^1$ and $Ar^2$ are 2, 3 or 4-pyrido, phenyl or substituted phenyl;

Y is aminocarbonyl or cyano;

x is 2 to 5;

R is H or loweralkyl;

$R^1$ and $R^2$ are hydrogen, cycloalkyl, loweralkyl, and $R^2$ and $R^3$ taken with the adjacent nitrogen may form a heterocyclic residue (including their diastereoisomers when possible) and pharmaceutical salts;

have cardiac antiarrhythmic activity.

EP 0 178 946 A2

# 1-(AMINOALKYL)-α,α-DIARYL PYRROLIDINE-PIPERIDINE- AND HOMOPIPERIDINE- ACETAMIDES AND ACETONITRILES

The present invention relates to 1-(aminoalkyl)-α,α-diaryl-3-pyrrolidineacetamides (and acetonitriles) and 3 and 4-piperidine and homopiperidineacetamides and acetonitriles and their cardiac antiarrhythmic effects.

α,α-Diphenyl-3-pyrrolidineacetamides, acetonitriles and methanes having antiarrhythmic activity are disclosed in U.S. 4,002,766 which have the general formula:

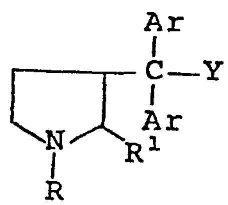

wherein R represents hydrogen, loweralkyl, lowercycloalkyl or phenyl-loweralkyl, $R^1$ represents hydrogen or loweralkyl, Ar represents phenyl and among the radicals disclosed for Y are carbamoyl and cyano. In contrast, while the pyrrolidine compounds in the present invention have similarly positioned carbamoyl or cyano radicals, the substituents at the one position are different than the prior art reference, in that a terminal amino group is present.

Methods of preparing starting hexahydro-1H-azepin-3-ol and -4-ols, the equivalent of 3 and 4-hydroxyhomopiperidines which are precursors to the homopiperidine compounds of this invention have been disclosed in the prior art (C.A. 64, 17567e and C.A. 53, 8160g, respectively).

Compounds of this invention have the formula:

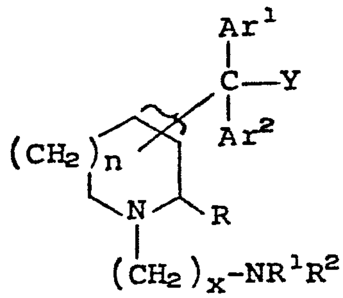

Formula I

wherein:

n is zero, one or two;

$Ar^1$ and $Ar^2$, which may be the same or different, are selected from 2-, 3- and 4-pyrido, phenyl and phenyl substituted by 1 to 3 radicals which may be the same or different and selected from loweralkyl, loweralkoxy, halogen or trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl or loweralkoxy and phenyl-loweralkyl wherein phenyl may be substituted by halogen, loweralkyl or loweralkoxy and $R^1$ and $R^2$ may be the same or different or, taken together with the adjacent nitrogen atom, form a heterocyclic residue selected from pyrrolidino, piperidino, 4-phenylpiperidino, 2,6-diloweralkyl-piperidino, 4-hydroxy-4-phenylpiperidino, 4-cyano-4-phenylpiperidino, piperazino, 4-loweralkyl-piperazino, 4-phenylpiperazino, (4-phenyl-loweralkyl)-piperazino and 4-morpholino radicals, or a pharmaceutically acceptable acid addition salt thereof.

A number of compounds within the above formula exist in different diastereoisomers; all such isomers are within the scope of the invention. Preferred

compounds include those in which, independently:

$Ar^1$ and $Ar^2$ both represent phenyl groups;

x is 2 or 3; and

$R^1$ and $R^2$ both represent loweralkyl groups or, together with the adjacent nitrogen atom, form a pyrrolidino residue.

In the further definition of symbols in the formulae hereof and where they appear elsewhere throughout this specification and claims, the terms have the following significance:

The term "loweralkyl" as used herein includes straight and branched chain radicals of up to eight carbons inclusive and is exemplified by such groups as methyl, ethyl, propyl, isopropyl, butyl, sec-butyl, amyl, isoamyl, hexyl, heptyl and octyl radicals and the like. The term "loweralkoxy" has the formula "O-loweralkyl".

The term "lowercycloalkyl" as used herein includes primarily cyclic alkyl radicals containing 3 to 9 carbon atoms inclusive and includes such groups as cyclopropyl, cyclobutyl, cyclopentyl, cyclohexyl, methylcyclohexyl, cycloheptyl and the like.

The terms "halo" or "halogen" when referred to herein includes fluorine, chlorine, bromine and iodine, preferably fluorine, chlorine and bromine.

Pharmaceutically acceptable acid addition salts are those salts formed by the compounds with any acid which is physiologically compatible in warm-blooded animals, such salts being formed by either strong or weak acids. Representative of strong acids are hydrochloric, hydrobromic, sulphuric and phosphoric acids. Representative of weak acids are fumaric, maleic, succinic, tartaric, oxalic, citric, hexamic and the like.

The compounds of Formula I are variously referred to herein as α,α-diarylacetamido (and acetonitrilo) heterocyclics.

The compounds of the present invention act to correct Ouabain-induced cardiac arrhythmias in animals as described more fully hereinbelow under pharmacology.

Cardiac arrhythmics in living animals can be treated by administering the α,α-diarylacetamido and acetonitriliheterocyclics of Formula I to a living animal body for cardiac arrhythmia effect in an effective amount to control arrhythmia as set forth hereinbelow under "Pharmaceutical Compositions and Administration". The acetamido compounds are preferred for their antiarrhythmic effect.

The invention therefore also relates to compounds of formula I, as defined above, for use in human or veterinary medicine, particularly cardiovascular medicine, and more particularly in treating cardiac arrhythmias.

The invention further relates to the use of a compound of formula I in the manufacture of an antiarrhythmic agent and to human and veterinary pharmaceutical compositions containing an effective amount of a compound of formula I and a pharmaceutically acceptable carrier therefor.

It is therefore an object of the present invention to provide novel 1-(aminoalkyl)-α,α-diaryl-3-pyrrolidine, piperidine and homopiperidineacetamides and acetonitriles which have utility in controlling cardiac arrhythmias in living animals and methods of preparation therefor and pharmaceutical compositions therewith.

Another object is to provide the 1-(aminoalkyl)-α,α-aryl-3-pyrrolidine, piperidine and homopiperidine

acetonitriles as chemical intermediates in the preparation of the more active acetamide derivatives.

Additional objects will become apparent to one skilled in the art and still others will become apparent hereinafter.

Preparation of compounds of Formula I and certain intermediates by two general methods is illustrated by schematic equations in Charts A and B.

## CHART A (Method A)

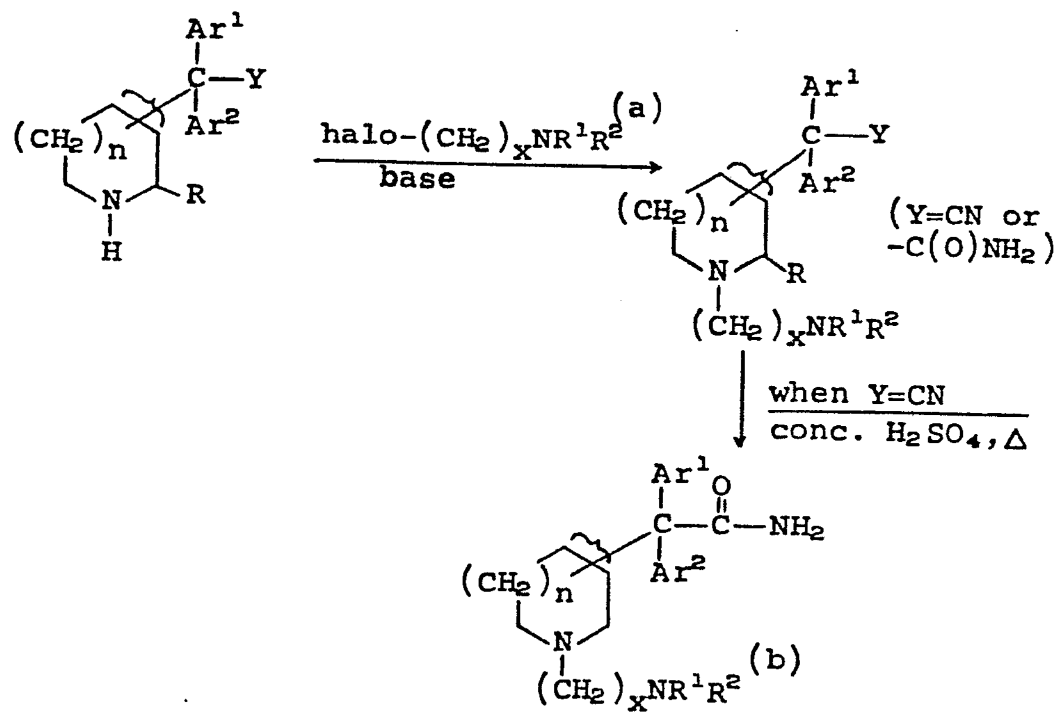


Footnotes Chart A:

(a) $R^1$ and/or $R^2$ cannot be hydrogen or unprotected piperazine. Otherwise, $R^1$ and $R^2$ include values under the definition of Formula I and, in addition, may be halo or form the following with the adjacent nitrogen: 1-phthalimido, 4-substituted piperazine such as 4-t-butoxycarbonylpiperazin-1-yl and

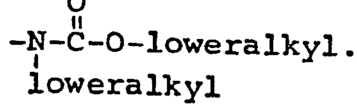

(b) $R^1$ and $R^2$ are the same as in Footnote a, except 4-t-butoxycarbonylpiperazin-1-yl is converted to piperazin-1-yl and

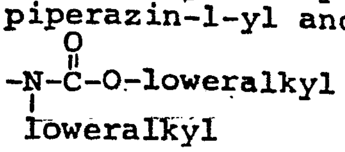 is converted to -NH-loweralkyl.

CHART B (Method B)

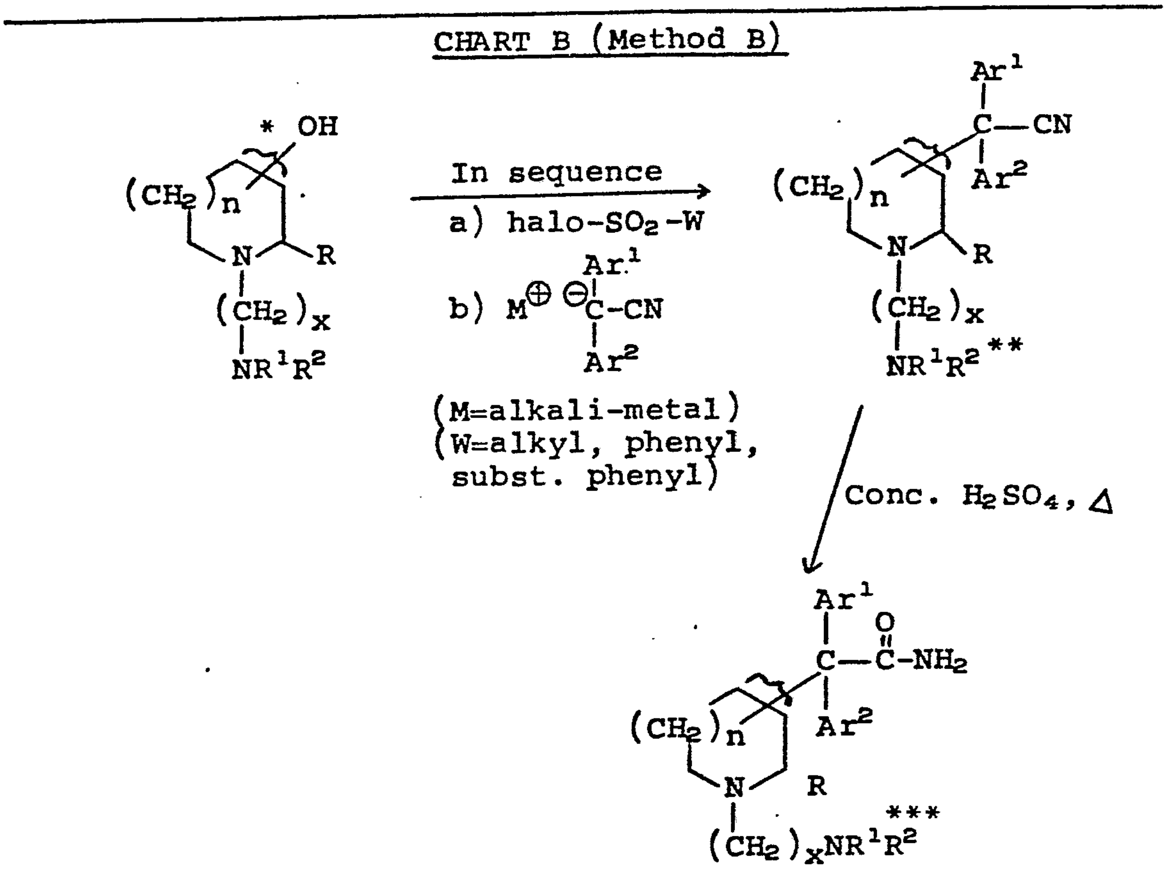


Footnotes Chart B:

*3-hydroxypyrrolidinyl and derivatives and 4-hydroxypiperidinyl and homopiperidinyl derivatives only; never 3 positioned piperidine or homopiperidine.

**same as footnote a) of Chart A.

***same as footnote b) Chart A.

Generally in Method A, the 1-unsubstituted-$\alpha,\alpha$-diarylacetamido (and acetonitrilo) heterocyclics are reacted with a haloalkylamino compound generally at reflux in a suitable non-reactive solvent. Solvents which are suitable range from alcohols and dimethylformamide to toluene. Generally, basic conditions facilitate the reaction. When the base is sodium hydride, an aprotic solvent is used. Addition of saturated sodium chloride during reaction generally enhanced the reaction. The product generally can be isolated by separating the organic phase and appropriately washing, separating and evaporating as illustrated in the examples and, if desired, converting to an acid addition salt and recrystallizing. When it is desirable to convert the resulting 1-alkylamino-$\alpha,\alpha$-diarylacetonitrilo heterocyclic to the $\alpha,\alpha$-diarylacetamido derivative, it is heated together with concentrated sulphuric acid and the product is isolated by a suitable means such as in Example 6.

Generally in Method B, the 3-hydroxy-1-alkylamino-heterocyclics, except 3-hydroxypiperidines and homopiperidines, are reacted with an alkane or arylsulphonyl chloride in a suitable solvent in the cold to obtain such as the 1-mesylated or 1-tosylated compound in the reaction mixture. To this mixture is then added the alkali-metal salt of $\alpha,\alpha$-diarylacetonitrile and the mixture is heated until reaction is complete. The product is separated by conventional means such as washing, extracting with acid solution and organic solvent and evaporating the solvent and, if desired, converting to an acid addition salt. As described for Method A, the nitrilo derivative may be converted to the acetamido derivative with concentrated sulphuric acid.

The primary amines of Formula I, wherein $R^1$ and $R^2$ are both hydrogen, are prepared from compounds wherein $-(CH_2)_x$-$\omega$-(1-phthalimido) derivatives as shown below by reacting with hydrazine hydrate, utilizing the method of Org. Syn. Coll. Vol.III, pp 151-153.

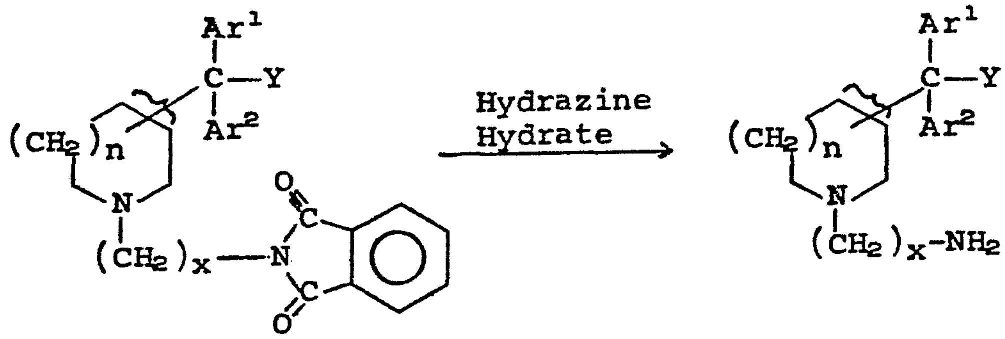


The monoalkylamines of Formula I wherein $R^1$ = methyl and $R^2$ = hydrogen may be prepared by reacting the primary amine derivatives prepared above with refluxing triethyl orthoformate for a period of time sufficient to form the methanimidic acid ester which is then reacted with sodium borohydride.

The monomethylamines may also be prepared by reaction of the primary amine with ethyl chloroformate and thereafter reducing with lithium aluminium hydride.

A further more generalized alternative for introduction of monoloweralkyl amine radicals is via the radical $-(CH_2)_x-\underset{\text{loweralkyl}}{N}-\overset{O}{\overset{\|}{C}}-O-$loweralkyl.

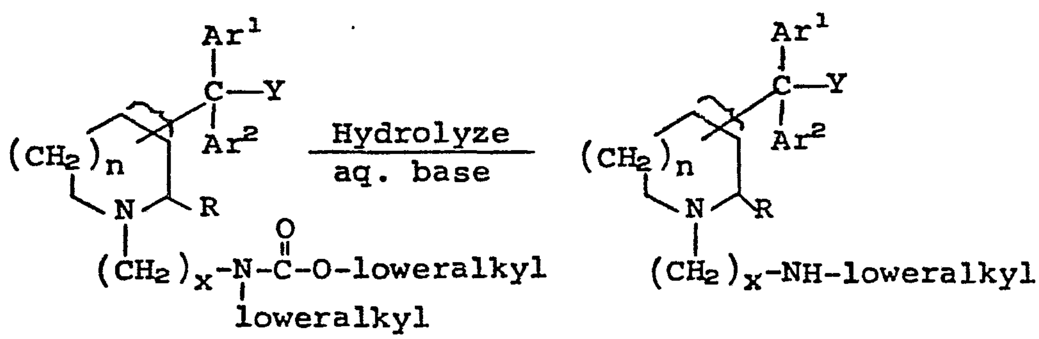


Other conversions of the primary amine of Formula Ic-1 to secondary, tertiary and heterocyclic amines are outlined in Chart C.

CHART C

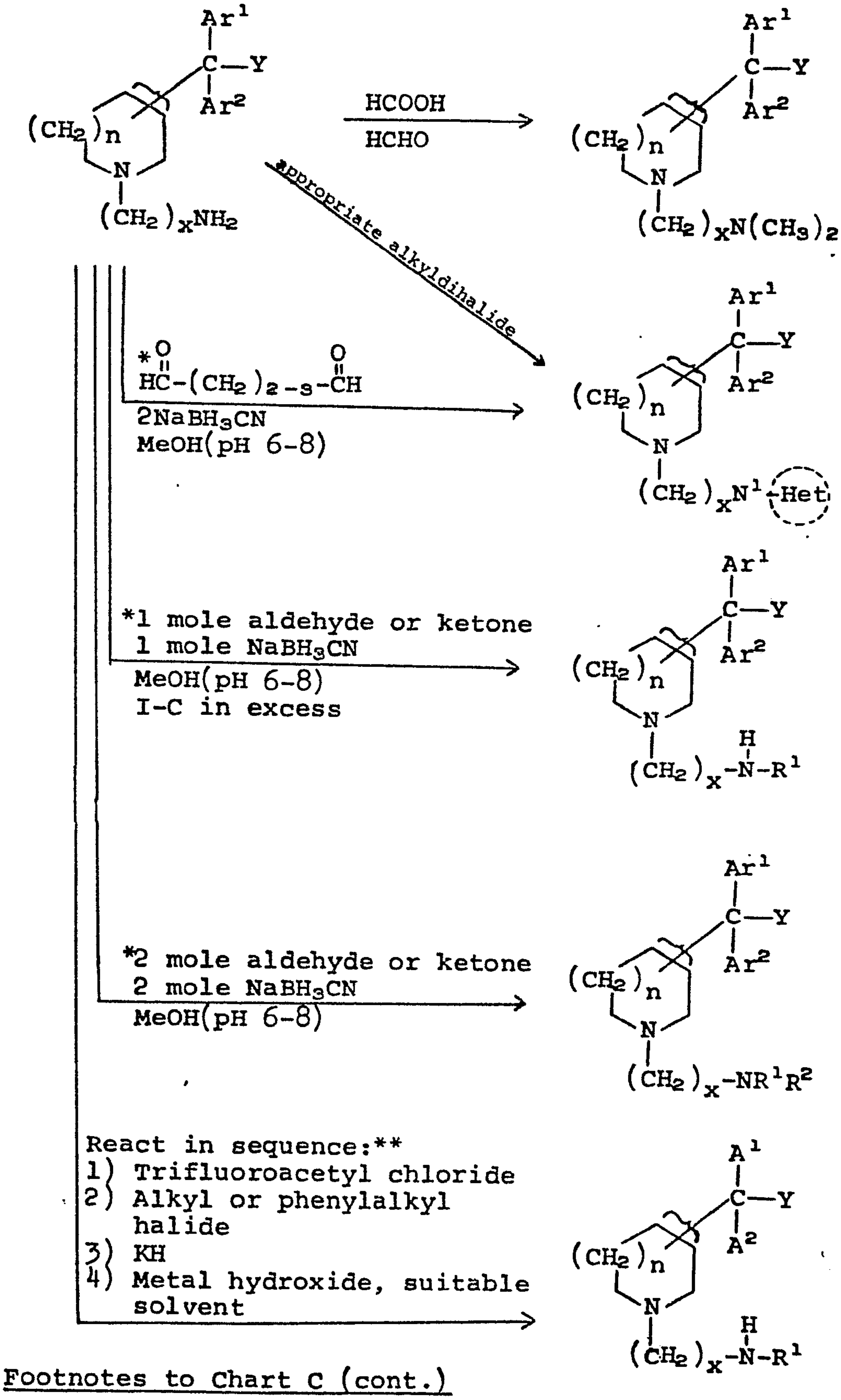


Footnotes to Chart C (cont.)

<u>Footnotes to Chart C:</u>

n = zero, one or two; Y = $-C{\equiv}N$ or $-\overset{O}{\overset{\|}{C}}-NH_2$.

*Method described by Borch, R. F. et al, J Amer. Chem. Soc. <u>93</u>, 2897 (1971).

Illustration of reaction of amino compound with aldehydes and ketones follows.

| Reactant | $NR^1R^2$ radical produced |
|---|---|
| 1) 1 mole acetaldehyde<br>1 mole $NaBH_3CN$<br>excess compound | $-NHC_2H_5$ |
| 2) 1 mole acetone<br>1 mole $NaBH_3CN$ | $-NHCH(CH_3)_2$ |
| 3) 1 mole benzaldehyde<br>1 mole $NaBH_3CN$<br>excess compound | $-NHCH_2C_6H_5$ |
| 4) 1 mole cyclohexanone<br>1 mole $NaBH_3CN$ | $-NHC_6H_{11}$. |
| 5) 2 mole acetaldehyde<br>2 mole $NaBH_3CN$ | $-N(C_2H_5)_2$ |
| 6) 1 mole acetaldehyde<br>1 mole $NaBH_3CN$<br>excess compound<br>followed by 1 mole HCHO<br>1 mole $NaBH_3CN$ | $-N(CH_3)(C_2H_5)$ |

**J. E. Norlander et al, Tetrahedron Letters 1978(50), pp 4987-4990

Acetamido compounds of Formula I wherein the $-NR^1R^2$ moiety is unsubstituted 4-piperazinyl are obtained by hydrolyzing a compound of Formula I wherein $-NR^1R^2$ is piperazino substituted (blocked) in the 4-position by an alkoxycarbonyl such as t-butoxycarbonyl, as obtained in Method A or B. Such compounds may also be obtained in Methods A or B in the second step by simultaneously hydrolyzing ($H_2SO_4$) acetonitrilo radical to the acetamido radical and the $-NR^1R^2$ which is a 4-alkoxycarbonylpiperazine radical to the unsubstituted 4-piperazinyl radical. Acetamido compounds of Formula I wherein $-NR^1R^2$ is -NH-loweralkyl may also be obtained in the second step of Methods A or B by simultaneous hydrolysis of the acetonitrolo radical to the acetamido radical and an $-NR^1R^2$ radical which is $-\underset{\text{loweralkyl}}{N}-\overset{O}{\overset{\|}{C}}-O$-loweralkyl to -NH-loweralkyl.

Compounds of Formula I are either isolated as the free base by evaporating or crystallizing or as an acid addition salt by reaction with the desired acid, using conventional means, crystallizing and recrystallizing from a suitable solvent or mixtures of solvents, usually an alcohol and an ester or ether.

Salts of compounds of Formula I may be converted to the free base by partitioning between a solvent such as methylene chloride and an aqueous base such as sodium hydroxide and evaporating the solvent layer *in vacuo*.

Broadly stated, a process of the invention encompassing both Methods A and B for preparing compounds of Formula I is comprised of the following steps:

Step 1, reacting a compound of the formula

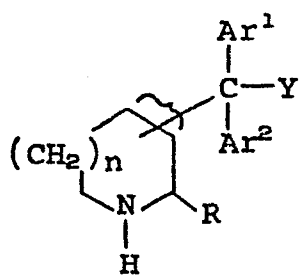

(II)

wherein R, $Ar^1$, $Ar^2$, Y and n have the values assigned under Formula I with a compound of the formula

$$\text{halo-}(CH_2)_x\text{-}NR^1R^2 \qquad (III)$$

wherein $R^1$, $R^2$ and x are as defined in Chart A to give a compound of the formula (as a free base or as a salt after reaction with an appropriate acid):

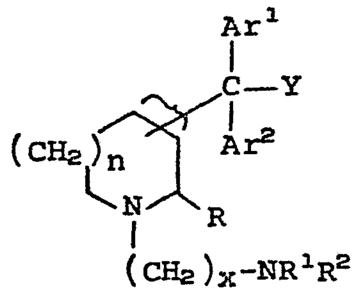

Formula I

or,

Step 2, alternatively reacting a compound of the formula

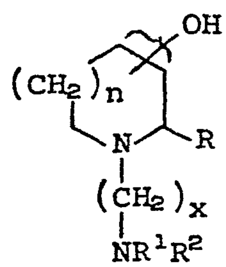

wherein R, $R^1$, $R^2$, x and n are as defined in Step 1 in sequence with compounds of the formulae

a) halo-$SO_2$W, and

b) 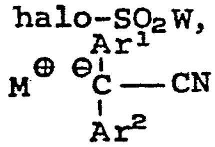

wherein W is loweralkyl, phenyl or phenyl substituted by non-interfering radicals, $M^+$ is an alkali-metal ion, and $Ar^1$ and $Ar^2$ are as defined under Formula I, to give a compound of the formula (as a free base or as a salt after reaction with an appropriate acid):

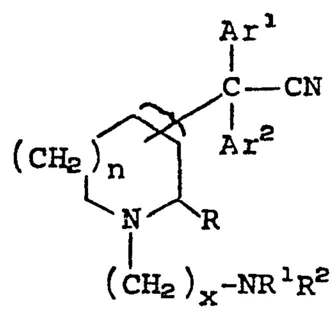

Step 3, optionally converting a compound of formula I so formed into another compound of formula I, for example subjecting a compound prepared in Step 1 wherein Y is CN or a compound prepared in Step 2 to hydrolyzing action of hot strong concentrated acid, preferably conc. sulphuric acid, to obtain a compound of the formula as free base or as a salt after reaction with an appropriate acid:

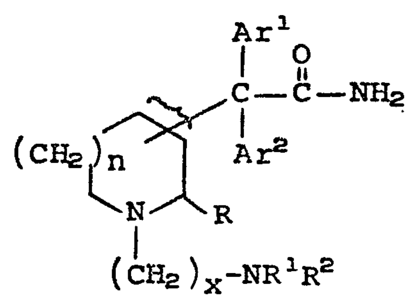

wherein R, $R^1$, $R^2$, $Ar^1$, $Ar^2$, x and n are as defined in Step 1.

A general method for the preparation of 1-unsubstituted-3 and 4-[α,α-diarylacetonitrilo and acetamido] pyrrolidines, piperidines and homopiperidines is outlined by schematic equation in Chart I.

CHART I

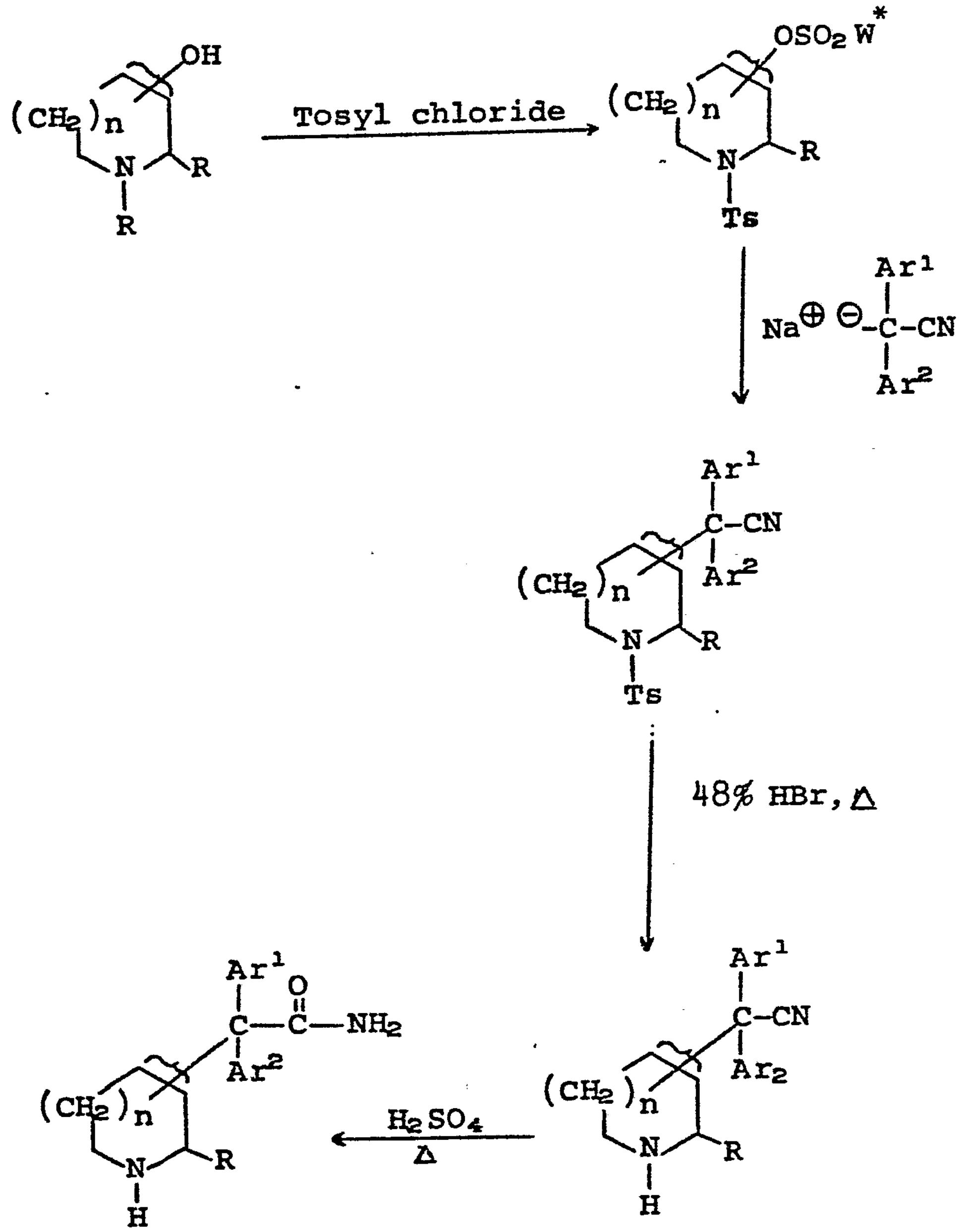

Another method for the preparation of 1-unsubstituted-3-[α,α-diarylacetonitrilo and acetamido]pyrrolidines and 1-unsubstituted 4-[α,α-diarylacetonitrilo and acetamido] piperidines and homopiperidines is outlined in Chart II.

CHART II

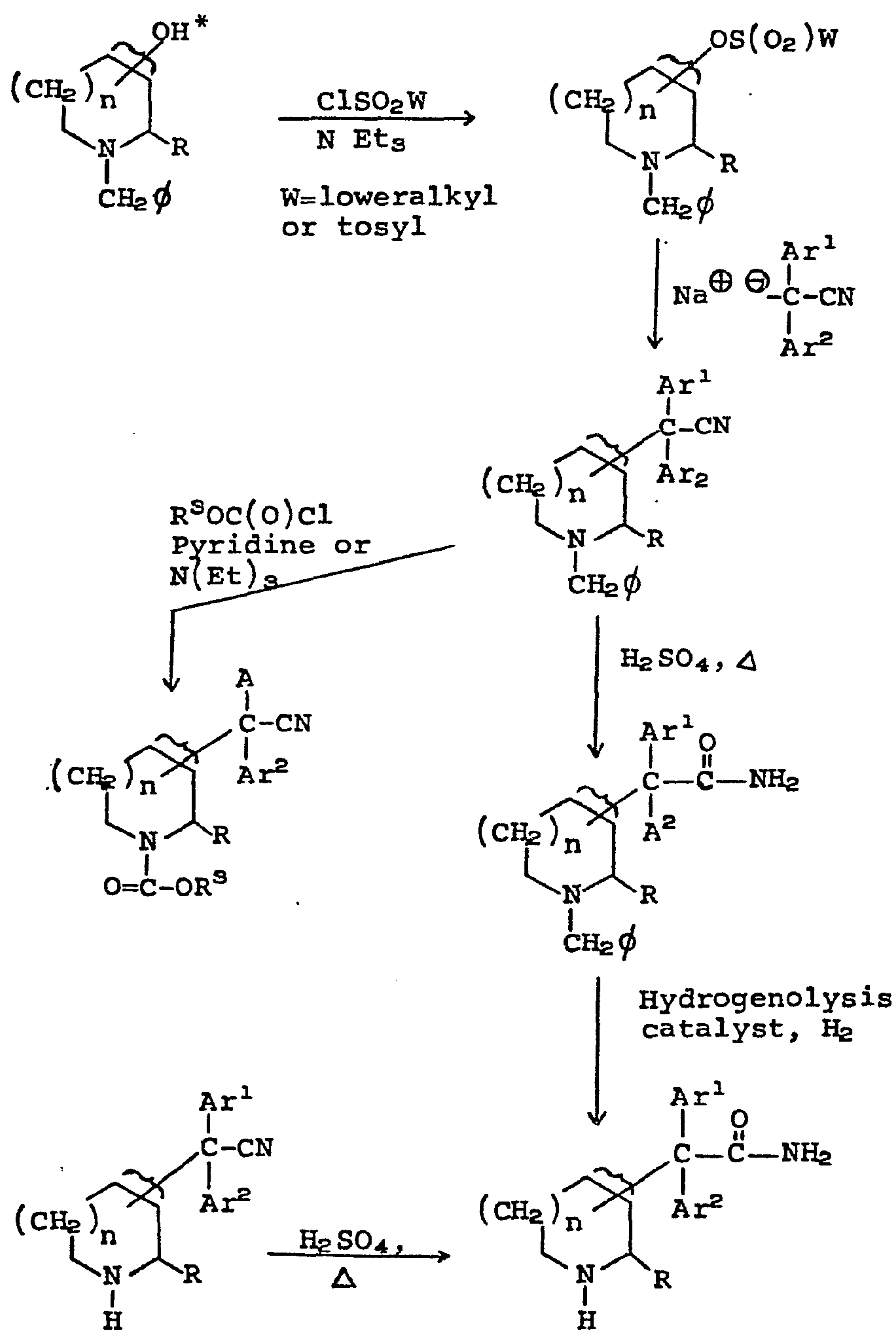


Footnote:
OH must be in 4-position when n = 1.

A general method for the preparation of 1-alkylamino-3-hydroxypyrrolidines and 4-hydroxypiperidines and 4-hydroxyhomopiperidines (excluding 3-hydroxypiperidines and 3-hydroxyhomopiperidines) is outlined by schematic equation in Chart III

CHART III

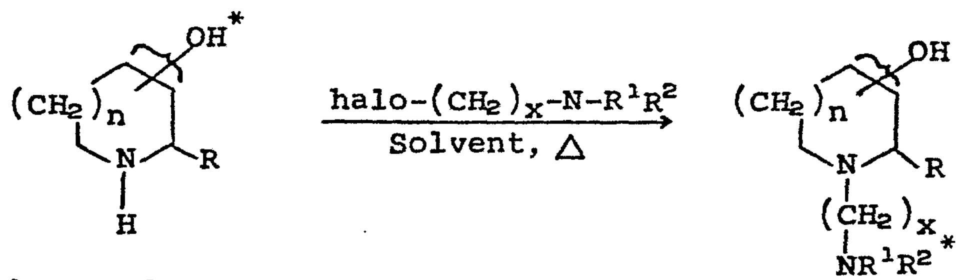


*$R^1$ and $R^2$ cannot be hydrogen.
**Excluding 3-hydroxypiperidine and 3-hydroxyhomopiperidine.

A method for the preparation of 1-alkylamino-3-hydroxypiperidine starting with malic acid used in Preparation 1 is outlined by schematic equation in Chart I.

CHART IV

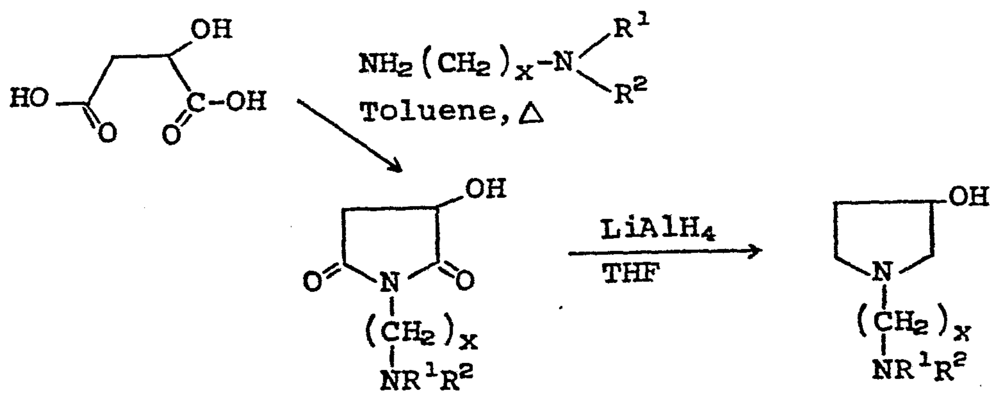


*$R^1$ and $R^2$ cannot be hydrogen.

As mentioned above, the prior art discloses methods of preparing 1-unsubstituted-hydroxyhomopiperidines which are used to prepare the homopiperidine derivatives of this invention.

The following preparations serve to illustrate the synthesis of certain precursors required in making the $\alpha,\alpha$-diarylacetonitrilo (and acetamido) heterocyclic compounds of Formula I but are not limiting as to scope. The preparation of certain 1-unsubstituted-$\alpha,\alpha$-diaryl-acetonitrilo and acetamido pyrrolidinyl precursors is taught in U. S. Patent 4,002,766 mentioned above.

### Preparation 1

1-[2-(Dimethylamino)ethyl]-1,5-dioxo-3-hydroxy-piperidine.

A solution of 40.2 g (0.30 mole) of malic acid and 26.40 g (0.30 mole) of N,N-dimethylethylenediamine in 200 ml of toluene was refluxed under a Dean Stark water separator for 18 hrs. The solution was cooled and the toluene layer was separated and concentrated to give 15 g of white needles (27%). The product was recrystallized from benzene-cyclohexane to give 11 g of the title product, m.p. 80-82.5°C. $^1$HNMR analysis confirmed the structure.

### Preparation 2

1-[2-(Dimethylamino)ethyl]-3-hydroxypyrrolidine.

A solution of 11.0 g (0.06 mole) of 1-[2-(dimethyl-amino)ethyl]-1,5-dioxo-3-hydroxypiperidine in 20 ml of tetrahydrofuran was added dropwise to a slurry of 4.56 g (0.12 mole) of lithium aluminium hydride in 100 ml of tetrahydrofuran. The solution was refluxed for 2 hr and cooled. A solution of 10% sodium hydroxide was added dropwise until a white granular precipitate formed. The mixture was filtered and the filter cake washed with tetrahydrofuran. The filtrate and wash were combined and concentrated. The residue was distilled under high vacuum to yield 4.8 g (50%), the title product as an oil, b.p. (~0.5 mm) 106-110°C. $^1$HNMR confirmed the structure.

## Preparation 3

**1-[(4-Methylphenyl) sulphonyl]-3-piperidinol-4-methylphenylsulphonate ester.**

A solution of 73.5 g (0.728 mole) of 3-hydroxypiperidine and 350 g (1.84 mole) of p-toluenesulphonyl chloride in 1 litre of pyridine was stirred at room temperature for 17.5 hr. The solution was quenched in 1 litre of water. The aqueous mixture was extracted with several portions of methylene chloride and the combined methylene chloride layers were extracted with several portions of 1 M sulphuric acid and then with several portions of 1 M sodium hydroxide. The organic solution was dried over magnesium sulphate and the solvent was removed *in vacuo* to give an oil residue. Crystalline product was obtained from the oil residue using the solvent pair diethyl ether-methylene chloride, m.p. 132-133°C.

| | | | |
|---|---|---|---|
| Analysis: | Calculated for $C_{19}H_{23}NO_5S_2$ | : C,55.73; | H,5.66; N,3.42 |
| | Found | : C,55.79; | H,5.69; N,3.38 |

## Preparation 4

**1-[2-(Dimethylamino)ethyl]-3-pyrrolidinol dimaleate.**

To a mixture of 8.59 g (0.22 mole) of crushed sodium hydroxide pellets and 30.97 g (0.22 mole) of 2-dimethylaminoethyl chloride hydrochloride in 600 ml of ethanol was added 18.68 g (0.22 mole) of 3-pyrrolidinol. The mixture was refluxed for 16 hr, cooled and filtered. The ethanol was evaporated from the filtrate *in vacuo* and the residue was partitioned between chloroform and dilute aqueous sodium hydroxide. The methylene chloride layer was dried and concentrated to give the free base of the title compound. The residue was reacted with maleic acid to give the dimaleate salt which was crystallized from isopropyl alcohol and recrystallized from isopropanol water to give 24.2 g (28%) of title product, m.p. 169-172°C.

Analysis: Calculated for $C_{16}H_{26}N_2O_8$: C,49.23; H,6.71; N,7.18

Found : C,49.11; H,6.61; N,7.08

Preparation 5

3-(Cyanodiphenylmethyl)-1-pyrrolidinecarboxylic acid, methyl ester.

To a solution of 36.5 g (0.10 mole) of α-(1-benzyl-3-pyrrolidinyl)-α,α-diphenylacetonitrile and 8.7 g (0.11 mole) of pyridine in methylene chloride was added dropwise at 25°C., 10.4 g (0.11 mole) of methyl chloroformate. The solution was refluxed for 16 hr. To the refluxing solution was added 26.1 g (0.33 mole) of pyridine and 31.2 g (0.33 mole) additional methyl chloroformate. Heating at reflux was continued for 5 hr. The mixture was cooled and washed four times with water. The methylene chloride layer was dried and concentrated in vacuo. The residual oil was crystallized twice from a mix of petroleum ether (30-60°) and isopropanol to give 22 g (68%) of title product, m.p. 133-135°C.

Analysis: Calculated for $C_{20}H_{20}N_2O_2$: C,74.98; H,6.29; N,8.74

Found : C,75.03; H,6.44; N,8.66

Preparation 6

α,α-Diphenyl-3-pyrrolidineacetamido oxalate.

A stirred solution of 22.0 g (0.060 mole) of α,α-diphenyl-3-pyrrolidineacetonitrile in 100 ml of conc. sulphuric acid was heated at 70°C. for 24 hr and then cooled. The cold reaction mixture was poured over ice and basified with 50% sodium hydroxide and ice. The mixture was extracted with chloroform. The chloroform extract was washed with water, dried over sodium sulphate and concentrated in vacuo to give the free base of the title compound. The free base was reacted with oxalic acid and crystallized from isopropyl alcohol to give 15 g of title product, m.p. 216-218.5°C.

## Preparation 7

**3-(Cyanodiphenylmethyl)-1-pyrrolidinecarboxylic acid, phenyl ester.**

To a solution of 47 g (0.13 mole) of α-(1-benzyl-3-pyrrolidinyl)-α,α-diphenylacetonitrile and 10.58 g (0.13 mole) of pyridine in 400 ml of methylene chloride was added, dropwise at 0°C., 62.7 g (0.39 mole) of phenyl chloroformate. The reaction mixture was refluxed for 16 hr. The mixture was cooled and washed 3 times with water and twice with dilute sodium hydroxide solution. The methylene chloride layer was then dried, filtered and concentrated *in vacuo*. The resulting solid was recrystallized twice from ethyl acetate to give 23 g (50%) of title compound, m.p. 154-155°C.

Analysis: Calculated for $C_{25}H_{22}N_2O_2$: C,78.51; H,7.32; N,5.79

Found : C,78.31; H,7.43; N,5.74

## Preparation 8

**α,α-Diphenyl-3-pyrrolidineacetamide.**

A solution of 3.0 g (0.008 mole) of 3-(cyanodiphenylmethyl)-1-pyrrolidinecarboxylic acid phenyl ester in 100 ml of 90% sulphuric acid was stirred at 70°C. for 20 hr. The reaction mixture was cooled, poured over ice and basified by adding, alternately, 50% sodium hydroxide and ice. The mixture was extracted with chloroform. The chloroform extract was washed with water, dried over sodium sulphate and concentrated *in vacuo*. The residue was crystallized in ethyl acetate. Recrystallization from ethyl acetate-ethanol gave 1.4 g (65%) of title product, molecular ion at 281 by mass spectrometer analysis.

## Preparation 9

**α,α-Diphenyl-3-pyrrolidineacetonitrile oxalate [1:1].**

**Preparation of Lithium n-propyl-Mercaptide Reagent**

To a mixture of 8.9 g (1.12 mole) of lithium hydride in 350 ml of hexamethylphosphoramide under a nitrogen atmosphere was added, dropwise at 10°C., 78.65 g (1.03

mole) of n-propanethiol. Stirring was continued for 3 hr and the mixture was filtered under nitrogen atmosphere. The reagent was titrated with 0.1 N hydrochloric acid to a phenolphthalein end point. The normality of the reagent was 0.74 N.

To 320 ml (0.24 mole) of the foregoing lithium n-propyl mercaptide reagent was added, portionwise at 25°C., 14 g (0.044 mole) of 3-(cyanodiphenylmethyl)-1-pyrrolidinecarboxylic acid, methyl ester. Stirring was continued at 25°C. for 16 hr and then at 50-60°C. for 2 hr. The mixture acidified with 6 N hydrochloric acid and then stirred at 60-70°C. for 20 min. The mixture was cooled and extracted 3 times with isopropyl ether. The aqueous layer was separated, basified with dilute sodium hydroxide and extracted 3 times with isopropyl ether. These last ether extracts were washed 3 times with water, dried and concentrated _in vacuo_. The residue was reacted with oxalic acid crystallizing the oxalate salt from isopropanol-water to give a 64% yield of the title compound. A portion was recrystallized from isopropanol-water, m.p. 181-184°C. (with decomposition).

Analysis: Calculated for $C_{20}H_{20}N_2O_4$: C,68.17; H,5.72; N,7.95

Found : C,68.05; H,5.68; N,7.93

## Preparation 10

### α,α- Diphenyl-1-phenylmethyl-3-pyrrolidineacetamide.

To 250 ml of concentrated sulphuric acid under agitation was added, slowly at 70°C., 170 g (0.48 mole) of α-(1-benzyl-3-pyrrolidinyl)-α,α-diphenylacetonitrile. Stirring was continued for 16 hr at 75-80°C. The solution was cooled and poured into a mixture of ice, 50% sodium hydroxide solution and chloroform. The chloroform layer was separated and the aqueous phase extracted three more times with chloroform. The combined chloroform layers were dried and concentrated _in vacuo_ to give 188 g of residual oil. A portion of the oil was crystallized in isopropanol and the solid recrystallized from a mix of

isopropanol and isopropyl ether to give a light brown solid, m.p. 135-138°C.

Analysis: Calculated for $C_{25}H_{26}N_2O$: C,81.05; H,7.07; N,7.56

Found : C,80.95; H,7.21; N,7.59

## Preparation 11

α,α-Diphenyl-3-pyrrolidineacetamide maleate [1:1].

A solution of 1.13 g of 1-benzyl-α,α-diphenyl-3-pyrrolidineacetamide in 50 ml of methanol was subjected to hydrogen (over 0.5 g of 10% palladium-on-charcoal catalyst) at 75°C. overnight in a Parr hydrogenation apparatus. The mixture was filtered and the filtrate was concentrated to give 0.783 g (80%) free base of the title compound as light tan gum. The mass spectra and infrared spectra were consistent with the structure. To a methanol solution of a portion of the free base was added a methanolic solution of maleic acid. The solution was concentrated to remove methanol and the residue crystallized. The solid was recrystallized twice from isopropanol-diethyl ether. The solid was dried at 110°/0.1 mm for 3 hr. The product liquified over a range of 110-145°C.

Analysis: Calculated for $C_{22}H_{24}N_2O_5$: C,66.65; H,6.10; N,7.07

Found : C,66.79; H,6.05; N,7.04

## Preparation 12

α,α-Diphenyl-3-pyrrolidineacetamide N-cyclohexylsulphamate hydrate [1:1].

A methanolic solution of 1.15 g of α,α-diphenyl-3-pyrrolidineacetamide and 0.735 g of hexamic acid was prepared and the methanol was evaporated from the solution to give a crystalline residue. The residue was recrystallized from ethanol, m.p. 103-106°C.

Analysis: Calculated for $C_{48}H_{72}N_6O_{11}$: C,59.24; H,7.46; N,8.64

Found : C,58.97; H,6.98; N,8.51

## Preparation 13

α,α-Diphenyl-1-(phenylmethyl)-4-piperidineacetonitrile hydrochloride.

To a prewashed slurry of 8.0 g (0.19 mole) of 57% sodium hydride in 300 ml of dimethylsulphoxide was added 32.8 g (0.17 mole) of diphenylacetonitrile. The solution was heated at 65°C. for 1 hr, during which time the solution developed a deep red colour. To the reaction mixture was added 55.90 g (.16 mole) of 1-(phenylmethyl)-4-piperidinol ester with 4-methylbenzenesulfonic acid in 50 ml of dimethylsulfoxide and the solution was stirred overnight at 60°C. The solution was cooled and poured into 1 litre of water. The aqueous solution was extracted three times with 150 ml portions of toluene. The toluene extracts were combined and 500 ml of 1N sulphuric acid was added. A gummy residue precipitated which was separated and partitioned with methylene chloride and 10% aqueous sodium hydroxide. The aqueous layer was extracted with methylene chloride and the combined methylene chloride extracts were dried over magnesium sulphate and concentrated to give 35.0 g (57%) of free base of the title compound as tan solid, m.p. 138-142°C. A portion of the free base in methanol was reacted with ethereal hydrogen chloride to give the hydrochloride salt, m.p. >250°C.

| | | | |
|---|---|---|---|
| Analysis: | Calculated for $C_{26}H_{27}ClN_2$: | C,77.50; | H,6.75; N,6.95 |
| | Found: | C,77.09; | H,6.76; N,7.04 |

## Preparation 14

α,α-Diphenyl-1-[4-(methylphenyl) sulphonyl]-3-piperidine-acetonitrile.

To a slurry of 0.15 g (0.012 mole) of prewashed sodium hydride in 100 ml of toluene was added 2.10 g (0.011 mole) of diphenylacetonitrile. The solution was refluxed for 2 hrs and 4.0 g (0.010 mole) of 1-[(4-methylphenyl)sulfonyl]-3-piperidinol-(4-methylphenyl)sulphonate ester in 100 ml of toluene was added. The solution was refluxed for 18 hr,

cooled, washed three times with 100 ml portions of water and dried over magnesium sulphate. The solution was evaporated to a gummy residue which was crystallized from absolute ethanol to give 2.50 g (58%) of a pale yellow powder, m.p. 135-136°C.

Analysis: Calculated for $C_{19}H_{26}N_2O_2S$: C,72.53; H,6.09; N,6.51

Found : C,72.79; H,6.10; N,6.52

Preparation 15

α α-Diphenyl-3-piperidineacetonitrile hydrochloride.

A solution of 5.00 g (0.012 mole) of α,α-diphenyl-1-[4-(methylphenyl)sulfonyl]-3-piperidineacetonitrile, 60 ml of 48% hydrobromic acid and about 5 g phenol (excess) was refluxed for 3.5 hr. The solution was cooled, poured onto ice and made basic with an excess of 50% sodium hydroxide. The mixture was extracted with three 150 ml portions of methylene chloride. The combined methylene chloride layers were dried and concentrated to give a black oily residue. The residue was taken up in 1:1 isooctane/toluene solution and the mixture was extracted with 10% aqueous hydrochloric acid solution. The acidic extract was made basic with 10% sodium hydroxide and extracted with methylene chloride. The extracts were dried over magnesium sulfate and concentrated to give 1.70 g (84%) of the free base of the title compound as a glassy residue. The free base was dissolved in isopropyl alcohol and reacted with ethereal hydrogen chloride to give the hydrochloride salt. The salt was recrystallized from ethanol/ethyl acetate to give 1.79 g (48%) of the title product, m.p. 171-177°C.

Analysis: Calculated for $C_{19}H_{21}ClN_2$: C,72.95; H,6.77; N,8.95

Found C,72.64; H,6.73; N,8.90

## Preparation 16

4-Cyanodiphenylmethyl)-1-piperidinecarboxylic acid phenyl ester.

To a solution of 34.0 g (0.093 mole) of α,α-diphenyl-1-(phenylmethyl)-4-piperidineacetonitrile in 300 ml of methylene chloride was added dropwise to 15.70 g (0.10 mole) of phenylchloroformate in 100 ml of methylene chloride. The solution was stirred 3 hr at room temperature, after which 11.10 g (0.11 mole) of triethylamine was added. The solution was stirred additionally for 1 hr, washed with 200 ml of water followed by 100 ml of 10% hydrochloric acid and dried over magnesium sulphate. The methylene chloride layer was concentrated to give a tan colored paste. The paste was recrystallized from isopropyl alcohol to give 19.70 g (54%) of solid. A portion of the solid was recrystallized from isopropyl alcohol, m.p. 141-143°C.

Analysis: Calculated for $C_{26}H_{24}N_2O_2$: C,78.76; H,6.10; N,7.06

Found : C,78.38; H,6.27; N,7.01

## Preparation 17

α,α-Diphenyl-4-piperidineacetamide fumarate monohydrate.

A solution of 18.50 g (0.046 mole) of 4-(cyanodiphenylmethyl)-1-piperidinecarboxylic acid phenyl ester in 100 ml of 90% sulphuric acid was heated at 65°C. for 18 hr and 90°C. for 6 hr. The mixture was cooled, poured onto ice and made basic with excess 50% sodium hydroxide. The aqueous solution was extracted with three 150 ml portions of chloroform. The combined chloroform extract was dried over magnesium sulphate and concentrated to give a residue which crystallized on standing. Recrystallization of the residue from ethanol/ethyl acetate gave 6.10 g (45%) of the free base of the title compound. A 1 g portion was reacted with fumaric acid, crystallizing from methanol-diethyl ether to yield 1.0 g of the salt, m.p. 172-175°C. (softens at 158°C.).

Analysis: Calculated for $C_{23}H_{26}N_2O_6$: C,64.47; H,6.59; N,6.54
Found : C,64.76; H,6.13; N,6.55

Preparation 18

α,α-Diphenyl-3-piperidineacetamide hemifumarate hemihydrate.

A solution of 2.3 g (0.0086 mole) of crude α,α-diphenyl-3-piperidineacetonitrile hydrochloride in 50 ml of 90% sulphuric acid was heated at 80°C. for 18 hr. The solution was cooled, made basic with excess 50% sodium hydroxide and extracted with methylene chloride. The extract was dried over magnesium sulphate and concentrated to yield 1.5 g (59%) of crude free base of the title compound. The fumarate was prepared in methanol using 0.5 equivalent of fumaric acid and adding diethyl ether to precipitate and give 0.50 g (16%) of title product, m.p. >250°C.

Analysis: Calculated for $C_{21}H_{25}N_2O_{3.5}$: C,69.78; H,6.97; N,7.75
Found : C,69.75; H,6.90; N,7.68

Preparation 19

1-(Phenylmethyl)-4-piperidinol ester with 4-methylbenzenesulphonic acid maleate [1:1].

A solution of 100 g (0.524 mole) of N-benzyl-4-hydroxypiperidine and 13 g (0.684 mole) of tosylchloride in 600 ml of pyridine was stirred at room temperature overnight. One litre of methylene chloride and 500 ml of 0.5 M aqueous sodium hydroxide were added to the reaction mixture. The reaction mixture was stirred for 10 min and the phases were separated. The methylene chloride layer was extracted with several portions of dilute sodium hydroxide, dried over magnesium sulphate and evaporated in vacuo to give an oil, the free base of the title compound. The free base was converted to the maleate salt which was recrystallized from methylene chloride-diethyl ether to give white crystalline solid, m.p. 159-160°C.

Analysis: Calculated for $C_{23}H_{27}NO_7S$: C,59.86; H,5.90; N,3.04
Found : C,59.79; H,5.86; N,2.95

Preparation 20 a and b

Following the procedure of Preparation 3,
3-hydroxyhomopiperidine, and
4-hydroxyhomopiperidine
are reacted with p-toluenesulphonyl chloride to give the following:

a) 1-[(4-methylphenyl)sulphonyl]-3-homopiperidinol-4-methylphenylsulphonate ester hydrochloride, and

b) 1-[(4-methylphenyl)sulphonyl]-4-homopiperidinol-4-methylphenylsulphonate ester hydrochloride.

Preparation 21 a and b

Following the procedure of Preparation 13,
1-[(4-methylphenyl)sulphonyl]-3-homopiperidinol-4-methylphenylsulphonate ester, and
1-[(4-methylphenyl)sulphonyl]-4-homopiperidinol-4-methylphenylsulphonate ester
are reacted with sodium hydride and diphenylacetonitrile to give the following:

a) α,α-diphenyl-1-[4-(methylphenyl) sulphonyl]-3-homopiperidinolacetonitrile, and

b) α,α-diphenyl-1-[4-(methylphenyl) sulphonyl]-4-homopiperidinolacetonitrile.

Preparation 22 a and b

Following the procedure of Preparation 15,
α,α-diphenyl-1-[4-(methylphenyl) sulphonyl)-3-homopiperidinolacetonitrile, and
α,α-diphenyl-1-[4-(methylphenyl) sulphonyl)-4-homopiperidinolacetonitrile
are subjected to refluxing 48% hydrobromic acid in phenol to give the following:

a) α,α-diphenyl-3-homopiperidineacetonitrile,and

b) α α-diphenyl-4-homopiperidineacetonitrile

which may be isolated as hydrochloride salts as in Preparation 13.

Preparation 23 a and b

Following the procedure of Preparation 18, α,α-diphenyl-3-homopiperidineacetonitrile and α,α-diphenyl-4-homopiperidineacetonitrile are hydrolyzed with conc. sulphuric acid (90%) to give the following:

a) α,α-diphenyl-3-homopiperidineacetamide, and
b) α,α-diphenyl-4-homopiperidineacetamide.

Preparation 24

α-(4-Chlorophenyl)-α-[1-(phenylmethyl)-3-pyrrolidinyl]-2-pyridineacetonitrile α isomer.*

To a solution of 136 g (0.77 mole) of 1-benzyl-3-pyrrolidinol and 78 g (0.79 mole) of triethylamine in 600 ml of dry benzene was added dropwise 8.86 g (0.77 mole) of methanesulphonyl chloride while cooling with an ice bath. The ice bath was removed and the mixture stirred 1 hr and filtered. The filter cake was washed twice with 100 ml of benzene.

In a separate flask, 175 g (0.77 mole) of α-(4-phenyl)-2-pyridylacetonitrile in 300 ml of dry toluene was added dropwise to a suspension of 40.3 g (0.84 mole) of 50% sodium hydride (mineral oil was removed by washing with ligroin) in 800 ml of dry toluene at 80°C. The mixture was heated to 80-85°C. for 1 hr and the benzene solution of 1-benzyl-3-pyrrolidinemethanesulphonate (prepared above) was added dropwise while maintaining the temperature. The solution was stirred for 2 hr at 85°C., cooled and extracted twice with water. The organic layer was dried over sodium sulphate and distilled. Yield 196 g (65%), b.p. 240-250°C./0.5 mm.

A sample (15 g) was chromatographed on the HPLC (Water Prep LC/system 500 A) using Prep Pak 500 silica column and isopropyl ether. An impurity was first to emerge followed by the two diastereomers which were partially separated. That portion of the first emerging isomer which was shown by T.L.C. (silica gel; ethyl acetate) to be pure was collected and concentrated. Yield 4 g (27%) of racemic mixture.

*Designated α-isomer as first emerging from the HPLC column.

Analysis: Calculated for $C_{24}H_{22}N_3Cl$: C,74.31; H,5.72; N,10.83
Found : C,74.38; H,5.69; N,10.96

Preparation 25

α-(4-Chlorophenyl)-α-[1-(phenylmethyl)-3-pyrrolidinyl]-2-pyridineacetonitrile β isomer.*

To a solution of 136 g (0.77 mole) of 1-benzyl-3-pyrrolidinol and 78 g (0.79 mole) of triethylamine in 600 ml of dry benzene was added dropwise 8.86 g (0.77 mole) of methanesulphonyl chloride while cooling with an ice bath. The ice bath was removed and the mixture stirred 1 hr and filtered. The filter cake was washed twice with 100 ml of benzene.

In a separate flask, 175 g (0.77 mole) of α-(phenyl)-2-pyridylacetonitrile in 300 ml of dry toluene was added dropwise to a suspension of 40.3 g (0.84 mole) of 50% sodium hydride (mineral oil was removed by washing with ligroin) in 800 ml of dry toluene at 80°C. The mixture was heated to 80-85°C. for 1 hr and the benzene solution of 1-benzyl-3-pyrrolidinemethanesulphonate (prepared above) was added dropwise while maintaining the temperature. The solution was stirred for 2 hr at 85°C., cooled and extracted twice with water. The organic layer was dried over sodium sulphate and distilled. Yield 196 g (65%), b.p. 240-250°C./0.5 mm.

A sample (15 g) was chromatographed on the HPLC (Water Prep LC/system 500A) using Prep Pak 500 silica column and isopropyl ether. An impurity was first to emerge followed by the two diastereomers which were partially separated. That portion of the second emerging isomer which was shown by T.L.C. (silica gel; ethyl acetate) to be pure was collected and concentrated. Yield 4.5 g (30%) of racemic mixture.

Analysis: Calculated for $C_{24}H_{22}N_3Cl_2$: C,74.31; H,5.72; N,10.83
Found : C,74.33; H,5.71 N,10.95

*Designated β-isomer as second emerging from the HPLC column.

## Preparation 26

3-[α-(4-Chlorophenyl)-α-(2-pyridinyl)-cyanomethyl]-1-pyrrolidinecarboxylic acid, phenyl ester α isomer and β isomer.

Following the procedure of Preparation 16, the title α isomer compound is obtained by reacting α-(4-chlorophenyl), α-[1-(phenylmethyl)-3-pyrrolidinyl]-2-pyridineacetonitrile α isomer (Preparation 24) with phenylchloroformate in pyridine-methylene chloride solvent.

The β isomer is prepared from the corresponding starting material α-(4-chlorophenyl), α-[1-(phenylmethyl)-3-pyrrolidinyl]-2-pyridineacetonitrile β isomer (Preparation 25).

## Preparation 27

α-(4-Chlorophenyl)-α-(2-pyridinyl)-3-pyrrolidineacetamide α isomer and β isomer.

Following the procedure of Preparation 17, the title α isomer compound is prepared by subjecting 3-[α-(4-chlorophenyl), α-(2-pyridinyl)-cyanomethyl]-1-pyrrolidinecarboxylic acid, phenyl ester α isomer (Preparation 26) to hydrolysis with hot 90% sulphuric acid.

The β isomer is prepared from the corresponding starting material: 3-[α-(4-chlorophenyl), α-(2-pyridinyl)-cyanomethyl]-1-pyrrolidinecarboxylic acid, phenyl ester β isomer (Preparation 26).

The following examples serve to illustrate the preparation of the novel compounds of Formula I, useful in compositions for treating arrhythmias in the method of treatment of this invention and/or in addition as in the instance of the nitriles as chemical intermediates in the preparation of the preferred acetamido derivatives. The scope of the invention as it pertains to the Formula I compounds is not, however, limited thereto.

Example 1

1-[2-(Dimethylamino)ethyl]-α,α-diphenyl-3-pyrrolidineacetonitrile dimaleate hydrate.

To a solution of 2.9 g (0.11 mole) of α α-diphenyl-3-pyrrolidineacetonitrile in dry toluene under a nitrogen blanket was added 0.58 g (0.012 mole) of 50% sodium hydride (in mineral oil suspension) and the mixture was refluxed for 2 hr. To the mixture was added at rapid drop under reflux conditions 1.6 g (0.011 mole) of 2-dimethylaminoethyl chloride in toluene. Reflux was continued for 18 hr. Water was added dropwise to the stirring reaction mixture while cooling to maintain a temperature of 25°C. When evolution of gas ceased, the toluene layer was separated, washed twice with water, dried over magnesium sulphate and concentrated. The residue, the free base of the title compound, was converted to the maleate salt and twice recrystallized from isopropyl alcohol-water to give 1.5 g (23%) of white solid, m.p. 153-157°C.

Analysis: Calculated for $C_{30}H_{37}N_3O_9$: C,61.74; H,6.39; N,7.20

Found : C,61.99; H,6.12; N,7.07

Example 2

1-[2-(Dimethylamino)ethyl]-α,α-diphenyl-3-pyrrolidineacetonitrile.

To a stirred solution of 3.0 g (0.019 mole) of 1-[2-(dimethylamino)ethyl]-3-pyrrolidinol and 2.32 g (0.023 mole) of triethylamine in 100 ml of toluene at 0°C. was added 2.40 g (0.021 mole) of methanesulphonylchloride. The mesylate solution was stirred 1 hr at 0°C. and filtered.

To a slurry of prewashed sodium hydride, 0.96 g (0.023 mole) in 200 ml of toluene at reflux was added 3.86 g (0.020 mole) of diphenylacetonitrile. The solution was refluxed for 1.5 hr during which time a tan-yellow precipitate formed and to this mixture was added the foregoing mesylate-toluene solution. The mixture was refluxed for an additional 4 hrs. and cooled, washed twice with 100 ml portions of water and extracted twice with 75 ml portions of 10% hydrochloric acid. The acidic aqueous wash was combined and extracted three times with 50 ml portions of methylene chloride. The combined methylene chloride extract was dried over magnesium sulphate and concentrated in vacuo to give 4.30 g (68%) of the title product, the molecular weight of which was confirmed by mass spectrometry analysis (M-W + 1 = 333).

## Example 3

Following the procedure of Example 1 and substituting the following for α,α-diphenyl-3-pyrrolidineacetonitrile

α,α-diphenyl-3-piperidineacetonitrile,
α,α-diphenyl-4-piperidineacetonitrile,
α,α-diphenyl-3-homopiperidineacetonitrile, and
α,α-diphenyl-4-homopiperidineacetonitrile,

there are obtained:

1-[2-(dimethylamino)ethyl]-α,α-diphenyl-3-piperidineacetonitrile,
1-[2-(dimethylamino)ethyl]-α,α-diphenyl-4-piperidineacetonitrile,
1-[2-(dimethylamino)ethyl]-α,α-diphenyl-3-homopiperidineacetonitrile, and
1-[2-(dimethylamino)ethyl]-α,α-diphenyl-4-homopiperidineacetonitrile.

## Example 4

1-[2-(Dimethylamino)ethyl]-α,α-diphenyl-3-pyrrolidineacetamide difumarate, monohydrate.

A mixture of 15 g (0.033 mole) of α,α-diphenyl-α-(3-pyrrolidinyl)acetamide hexamate [1:1], 4.7 g (0.033 mole) of 2-dimethylaminoethyl chloride hydrochloride and

2.6 g (0.066 mole) of sodium hydroxide in 100 ml of ethanol was stirred at reflux for 4.5 hr. A few milligrams of dilute sodium hydroxide were added and reflux was continued for 16 hr. Chloroform was added and the mixture was extracted three times with dilute hydrochloric acid. The aqueous layer was basified with 50% sodium hydroxide and extracted with isopropyl ether. The isopropyl ether extract was dried over magnesium sulphate and concentrated under reduced pressure. The concentrated ether was chromatographed on a 2.5" x 30" silica gel column, eluting with a mixture of 49% methanol, 49% chloroform and 2% ammonium hydroxide. The product (free base of the title compound in solution) was collected after eluting with 300 ml of the solvent mixture. The fumarate salt was prepared and crystallized from a mixture of isopropyl alcohol-isopropyl ether and recrystallized from isopropyl alcohol to give 1.69 g (8.5%) of the crystalline title compound, m.p. 162-165°C. with decomposition (and loss of crystalline structure at 120°C.

Analysis: Calculated for $C_{30}H_{39}N_3O_{10}$: C,59.89; H,6.53; N,6.98

Found : C,60.30; H,6.23; N,6.96

Example 5

1-[2-(Dimethylamino)ethyl]-α,α-diphenyl-3-pyrrolidine-acetamide difumarate, hemihydrate.

To a mixture of 8.5 g (0.21 mole) of crushed sodium hydroxide, 12.7 g (0.089 mole) of 2-dimethylaminoethyl chloride hydrochloride in ethanol was added an ethanolic solution of 22.50 g (0.061 mole) of α,α-diphenyl-α-(3-pyrrolidinyl)acetamide. The mixture was refluxed for 24 hr and cooled. The mixture was filtered and the filtrate was concentrated *in vacuo*. The residue was partitioned between chloroform and dilute sodium hydroxide. The chloroform layer was dried over magnesium sulphate and concentrated. The residue was dissolved in 100 ml of dilute hydrochloric acid and continuously extracted with chloroform for 30 hr. on evaporation of the chloroform layer, 6.5 g starting material was found. The aqueous

acid layer was basified and extracted three times with chloroform. The chloroform layer was dried and concentrated. The residue, the free base of the title compound, was reacted with fumaric acid and crystallized from isopropyl alcohol-isopropyl ether. Recrystallization from isopropyl alcohol and water gave 11.0 g (30%) of title compound which loses its crystalline structure and slowly decomposes over the range 123-187°C.

Analysis: Calculated for $C_{30}H_{38}N_3O_{9.5}$: C,60.80; H,6.46; N,7.09

Found : C,60.99; H,6.38; N,7.05

Example 6

1-[2-(Dimethylamino)ethyl]-α,α-diphenyl-3-pyrrolidineacetamide difumarate.

A solution of 3.30 g (0.01 mole) of 1-[2-(dimethylamino)ethyl]-α,α-diphenyl-3-pyrrolidineacetonitrile in 70 ml of 90% sulphuric acid was heated at 80°C. for 24 hr. The solution was cooled, poured onto ice and the resulting mixture was neutralized with 50% sodium hydroxide. The mixture was extracted three times with 70 ml portions of methylene chloride. The combined chloroform extract was dried over magnesium sulphate and concentrated to yield 2.1 g (60%) of crude free base of the title compound. The free base was reacted with 1.39 g (0.012 mole) of fumaric acid in methanol. Diethyl ether was added to the solution until the cloud point was reached and several drops of water were added to clear the solution. The solution was allowed to stand 3 hr and the resulting precipitate was separated by filtration and dried at 80°C. under high vacuum to yield 2.1 g (38%) of crystals confirmed to be the title product by $^1$HNMR analysis.

Example 7

1-[2-(Dimethylamino)ethyl]-α,α-diphenyl-3-pyrrolidineacetamide difumarate hemihydrate.

To a rapidly stirred solution prepared by mixing 4.8 litres of acetonitrile, 1.4 litres of 10% aqueous sodium hydroxide and 2.2 liters of saturated aqueous

sodium chloride solution was added 48.40 g (0.15 mole) of α,α-diphenyl-3-pyrrolidineacetamide hydrochloride and 32.40 g (0.23 mole) of dimethylaminoethyl chloride hydrochloride. The solution was stirred at room temperature for 18 hr. The layers were separated and the organic phase was concentrated without heating to about 500 ml volume. The organic phase concentrate was diluted with 1 liter of methylene chloride and extracted twice with 500 ml portions of 1 N sulphuric acid. The aqueous acidic extracts were washed with 200 ml of methylene chloride. The aqueous layer was made basic with excess 10% sodium hydroxide and extracted three times with 200 ml portions of methylene chloride. These latter methylene chloride extracts were combined and dried over magnesium sulphate and concentrated. The residue, the free base of the title compound, was reacted with 29.14 g (0.126 mole) of fumaric acid in 600 ml of methanol and diethyl ether was added until the cloud point was reached. One ml of water was added and methanol was added until the solution cleared. The salt precipitated on standing over night at ambient temperature and was separated and dried to give 52.40 g (60%). The salt was left open to the air for 18 hr. The salt softened at 170°C. with m.p. 177-179°C.

| | | |
|---|---|---|
| Analysis: Calculated for $C_{30}H_{29}N_3O_{8.5}$ | : C,60.82; | H,6.46; N,7.04 |
| Found | : C,60.60; | H,6.27; N,7.03 |

## Example 8

1-[3-(Dimethylamino)propyl]-α,α-diphenyl-3-pyrrolidineacetamide sesquifumarate, hemihydrate.

A solution of 7.00 g (0.025 mole) of α,α-diphenyl-3-pyrrolidineacetamide, 4.27 g (0.027 mole) of N,N-dimethylaminopropyl chloride hydrochloride and 10.6 g (0.010 mole) of sodium carbonate in 400 ml of dimethylformamide was stirred for 6 hr at 80°C. and for 12 hr at room temperature. The solution was concentrated and the concentrate was diluted with 250 ml of water and extracted three times with 70 ml portions of methylene chloride. The methylene

chloride extracts were combined and extracted twice with 70 ml portions of hydrochloric acid. The acidic extracts were made basic with 10% aqueous sodium hydroxide and extracted with methylene chloride. The methylene chloride layer was dried over magnesium sulphate and concentrated to give 6.10 g (67%) of free base (crude) of the title compound. The fumarate salt was prepared in methanol and crystallized by adding diethyl ether. Recrystallization of the salt from methanol-diethyl ether and exposing to the air for 18 hr gave the title compound as white powder, m.p. 182-184°C.

Analysis: Calculated for $C_{29}H_{33}N_3O_{7.5}$: C,63.49; H,6.98; N,7.66

Found : C,63.34; H,6.82; N,7.65

Example 9

1-[2-(Diethylamino)ethyl]-α,α-diphenyl-3-pyrrolidineacetamide difumarate.

A mixture of 10.00 g (0.032 mole) of α,α-diphenyl-3-pyrrolidineacetamide hydrochloride, 6.80 g (0.040 mole) of diethylaminoethyl chloride hydrochloride, 600 ml of acetonitrile, 120 ml of 1N sodium hydroxide and 240 ml of saturated aqueous sodium chloride was stirred rapidly for 18 hr at ambient temperature. The acetonitrile layer was removed. The aqueous solution was extracted twice with 200 ml of methylene chloride. The methylene chloride layer was extracted with two 200 ml portions of 1N sulphuric acid. The aqueous acidic layer was made basic with 1N sodium hydroxide and extracted three times with 150 ml portions of methylene chloride. The three methylene chloride extracts were combined and dried over magnesium sulphate and filtered. Concentration of the filtrate gave 11.60 g (95%) of crude free base of the title compound. The free base was reacted with 7.4 g of fumaric acid in methanol. Diethyl ether was added until the cloud point was reached. One ml of water was added and enough methanol was added to clear the solution. The solution was allowed to stand 48 hr and the salt was separated by

filtration. On drying at 56°C. under vacuum, 10.30 g (53%) of the title compound as white powder, m.p. 175-178°C. was obtained.

Analysis: Calculated for $C_{24}H_{33}N_3O$: C,62.83; H,6.76; N,6.87

Found : C,62.63; H,6.85; N,6.85

Example 10

α,α-Diphenyl-1-[2-(1-pyrrolidinyl)ethyl]-3-pyrrolidineacetamide.

A solution of 2.88 g (0.025 mole) of 1-(2-hydroxyethyl)pyrrolidine methanesulphonate in dry pyridine was combined with a solution of 11.42 g (0.025 mole) of α,α-diphenyl-α-(3-pyrrolidinyl)-acetamide in dry pyridine. The reaction mixture was stirred under reflux for 72 hr and thereafter concentrated in vacuo. The residue was partitioned between chloroform and dilute aqueous sodium hydroxide solution. The chloroform layer was concentrated in vacuo and the residue was chromatographed on a silica gel column by the gradient method eluting in the following manner:

| Solvent Composition (chloroform:methanol) | Volume |
|---|---|
| 100:0 | 3.0 litres |
| 99:1 | 4.0 " |
| 98:2 | 5.0 " |
| 96:4 | 5.5 " |
| 92:8 | 2.0 " |
| 88:12 | 2.5 " |
| 84:16 | 2.5 " |

The last 3.5 litres of eluent were combined and concentrated in vacuo. Mass spectrometry analysis showed the desired compound was present.

Example 11

1-[2-(Dimethylamino)ethyl]-α,α-diphenyl-3-piperidineacetamide difumarate, monohydrate.

A solution of 4.70 g (0.016 mole) of α,α-diphenyl-3-piperidineacetamide, 2.53 g (0.017 mole) of dimethylaminoethyl chloride hydrochloride and 4.22 g (0.048 mole) of sodium bicarbonate in 500 ml of dimethylformamide was heated at 70-80°C. for 3 hr. The solution was cooled and the DMF was removed by evaporating. The residue was diluted with 100 ml of water, extracted three times with 70 ml portions of methylene chloride. The combined methylene chloride extract was dried over magnesium sulphate and concentrated to give 4.6 g (79%) of the free base of the title compound as an oil. The free base was reacted with fumaric acid in methanol. The methanol was evaporated to leave a gummy residue. The residue was crystallized from ethyl alcohol-ethyl acetate to give 3.5 g (35%) of the title compound as white powder, m.p. 173-178°C.

Analysis: Calculated for $C_{31}H_{41}N_3O_{10}$: C,60.48; H,6.71; N,6.82
Found : C,60.68; H,6.46; N,6.40

Example 12

1-[2-(Dimethylamino)ethyl]-α,α-diphenyl-4-piperidineacetamide difumarate, hemihydrate.

To a rapidly stirred solution prepared by mixing 480 ml of acetonitrile, 140 ml of 10% aqueous sodium hydroxide and 220 ml of saturated sodium chloride solution was added 4.00 g (0.014 mole) of α,α-diphenyl-4-piperidineacetamide fumarate hydrate and 2.35 g (0.16 mole) of N,N-dimethylaminoethyl chloride hydrochloride. The free base of the title compound was isolated and reacted with fumaric acid as in Example 7 to give 3.0 g (35%) of the title compound, m.p. 231-232°C.

Analysis: Calculated for $C_{31}H_{40}N_3O_{9.5}$: C,61.37; H,6.65; N,6.93
Found : C,61.58; H,6.65; N,6.77

Example 13

1-[2-(Dimethylamino)ethyl]-α,α-diphenyl-2-methyl-3-pyrrolidineacetonitrile.

Following the procedure of Example 1, α,α-diphenyl-2-methyl-3-pyrrolidineacetonitrile in toluene is reacted with sodium hydride followed by 2-dimethylaminoethyl

chloride. Water is added and the free base is obtained by the same procedure as in Example 1.

Example 14

1-[2-(Dimethylamino)ethyl]-α,α-diphenyl-2-methyl-3-pyrrolidineacetamide.

Following the procedure of Example 6, 1-[2-(dimethylamino)ethyl]-α,α-diphenyl-2-methyl-3-pyrrolidineacetonitrile is hydrolyzed with concentrated (90%) sulphuric acid to give the title compound and thereafter isolated as in Example 6.

Example 15

Following the procedure of Example 10 but substituting the following for 1-(2-hydroxyethyl)pyrrolidine methanesulfonate:

1-(2-hydroxyethyl)piperidine methanesulphonate,
1-(2-hydroxyethyl)-4-phenylpiperidine methanesulphonate,
1-(2-hydroxyethyl)-2,6-dimethylpiperidine methanesulphonate,
1-(2-hydroxyethyl)-4-cyano-4-phenylpiperidine methanesulphonate,
1-(2-hydroxyethyl)-4-phenyl-1,2,3,6-tetrahydropiperidine methanesulphonate,
1-(2-hydroxyethyl)-4-methylpiperazine methanesulphonate,
1-(2-hydroxyethyl)-4-phenylmethylpiperazine methanesulphonate,
1-(2-hydroxyethyl)-4-morpholine methanesulphonate, and
1-(2-hydroxyethyl)-4-tertiarybutoxycarbonylpiperazine methanesulphonate,

there are obtained:

a) α,α-diphenyl-1-[2-(1-piperidinyl)ethyl]-3-pyrrolidineacetamide,

b) α,α- diphenyl-1-[2-(4-phenylpiperidin-1-yl)ethyl]-3-pyrrolidineacetamide,

c) α,α-diphenyl-1-[2-(2,6-dimethylpiperidin-1-yl)ethyl]-3-pyrrolidineacetamide,

d) α,α-diphenyl-1-[2-(4-cyano-4-phenylpiperidin-1-yl)ethyl]-3-pyrrolidineacetamide,

e) α,α-diphenyl-1-[2-(4-phenyl-1,2,3,6-tetrahydropiperidine-1-yl)ethyl]-3-pyrrolidineacetamide,

f) α,α-diphenyl-1-[2-(4-methylpiperazin-1-yl)ethyl]-3-pyrrolidineacetamide,

g) α,α-diphenyl-1-[2-(4-phenylmethylpiperizin-1-yl)ethyl]-3-pyrrolidineacetamide,

h) α,α-diphenyl-1-[2-(morpholin-4-yl)ethyl]-3-pyrrolidinacetamide, and

i) α,α-diphenyl-1-[2-(4-tertiarybutoxycarbonylpiperazin-1-yl)ethyl]-3-pyrrolidineacetamide.

## Example 16

### α,α-Diphenyl-1-[2-(1-piperazinyl)ethyl)-3-pyrrolidineacetamide.

The title compound is obtained by hydrolyzing α,α-diphenyl-1-[2-(4-tertiarybutoxycarbonylpiperazin-1-yl)ethyl]-3-pyrrolidineacetamide.

## Example 17

### 1-[2-(Dimethylamino)ethyl]-α-(4-chlorophenyl), α-(2-pyridinyl)-3-pyrrolidineacetamide, α isomer and β isomer.

Following the procedure of Example 5, the α and β isomers of α-(4-chlorophenyl), α-(2-pyridinyl)-3-pyrrolidine acetamide as prepared in Preparations 27a and b are reacted with 2-dimethylaminoethyl chloride to give the title compound.

## Pharmacology

The action of compounds of this invention in correcting cardiac arrhythmias or preventing cardiac arrhythmias is demonstrated by the following procedures:

### Ouabian Induced Arrhythmias

Correction of existing cardiac arrhythmias of ventricular origin is carried out on (1) adult mongrel dogs which are under barbiturate anaesthesia during the test. A Grass Model 7 Polygraph was used for recording femoral arterial blood pressure (Statham P23AC Transducer) and the electrocardiagram (Grass 7P4 Preamplifier). Ouabain was given intravenously in an initial dose of 40 µg/kg and in a second dose of 20 µg/kg 30 minutes after the first dose and in subsequent doses of 10 µg/kg which were repeated at 15 min. intervals as required for producing cardiac arrhythmias that persisted for at least 15 minutes. When the arrhythmias were established, the test compounds were administered by infusion (Harvard Model 942 Infusion Pump) into a femoral vein at a rate of 1 mg/kg/min. Concentration of compound was adjusted according to the weight of the dog to allow a volume infusion of 1 ml/min. The compound was considered to be active as antiarrhythmic agent if reversion to sinus rhythm occurred which was maintained for at least 30 minutes.

Data obtained for one preferred compound, namely 1-[2-(dimethylamino)ethyl]-α,α-diphenyl-3-pyrrolidine-acetamide as represented by its defumarate hemihydrate salt are shown in Table 1.

Table 1

Effect of compound of Example 5: 1-[2-(dimethylamino)-ethyl]-α,α-diphenyl-3-pyrrolidine acetamide on Cardiac Arrhythmia in Dogs.

| Arrhythmia Model | Correcting Dose mg/kg, i.v. (computed as free base) |
|---|---|
| Ouabain-Induced[1] | 5.0 |

[1]Cardiac arrhythmias produced by method of Lucchessi and Hardman, 1961., J. Pharmacol. Exp. Ther. 132, 372-381.

Pharmaceutical Compositions and Administration

The invention further provides pharmaceutical compositions for administration to a living animal body comprising, as active ingredients, at least one of the compounds according to the invention in association with a pharmaceutical carrier or excipient. The compounds are thus presented in a therapeutic composition suitable for oral, rectal, parenteral or intracardial administration. Thus, for example, compositions for oral administration are preferably solids and can take the form of capsules, tablets or coated tablets containing carriers conveniently used in the pharmaceutical art. Suitable tableting excipients include lactose, potato and maize starches, talc, gelatin and stearic and silicic acids, magnesium stearate and polyvinyl pyrrolidone.

For parenteral administration, the carrier or excipient can be a sterile, parenterally acceptable liquid; e.g., water, or a parenterally acceptable oil; e.g., arachis oil, contained in ampoules.

In compositions for rectal administration the carrier can comprise a suppository base; e.g., cocoa butter, or a glyceride.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount; i.e., such that a suitable effective dosage will be obtained consistent with the dosage form employed. The exact individual dosages, as well as daily dosages, will, of course, be determined according to standard medical principles under the direction of a physician or veterinarian. Generally, the pharmacology on animals suggests the oral dosage effective to correct arrhythmias will be about 3 times that of the intravenous dosage. The animal data also suggest dosage requirements

will be about half that of quinidine for the more active compounds.

Based on the animal data, allowing for variation in species and severity of cardiac arrhythmia, unit dosages containing an amount of compound equivalent to about 1 to about 100 mg/kg of body weight are contemplated. Based on all of the above considerations, a choice in a range of unit oral dosages for humans of about 10 to about 1000 mg is contemplated, preferably about 10 to 600 mg for a more active compound such as Example 5. Daily dosages of about 30 to 2400 mg are contemplated for humans and, obviously, several unit dosage forms may be administered at about the same time. However, the scope of the invention is not to be limited by these contemplations due to the uncertainty in transpositions discussed above.

Examples of unit dosage compositions are as follows:

Capsules

| | Ingredients | Per Cap. |
|---|---|---|
| 1. | Active ingredient | 10.0 mg. |
| 2. | Lactose | 146.0 mg. |
| 3. | Magnesium Stearate | 4.0 mg. |

Procedure:

1. Blend 1, 2 and 3.
2. Mill this blend and blend again.
3. This milled blend is then filled into #1 hard gelatin capsules.

Tablets (10 mg)

| | Ingredients | Mg./Tab. |
|---|---|---|
| 1. | Active ingredient | 10.0 mg. |
| 2. | Corn starch | 20.0 mg. |
| 3. | Kelacid | 20.0 mg. |
| 4. | Keltose | 20.0 mg. |
| 5. | Magnesium stearate | 1.3 mg. |

## Tablets (50 mg)

| Ingredients | Mg/Tab. |
|---|---|
| 1. Active ingredient | 50.0 mg. |
| 2. Milo starch | 20.0 mg. |
| 3. Corn starch | 38.0 mg. |
| 4. Lactose | 90.0 mg. |
| 5. Calcium stearate | 2.0 mg. |
| | 200.0 mg. |

Procedure:

1. Blend 1, 2, 3 and 4.
2. Add sufficient water portionwise to the blend from Step #1 with careful stirring after each addition. Such additions of water and stirring continue until the mass is of a consistency to permit its conversion to wet granules.
3. The wet mass is converted to granules by passing it through the oscillating granulator, using 8-mesh screen.
4. The wet granules are then dried in an oven at 140°F.
5. The dried granules are then passed through an oscillating granulator, using a 10-mesh screen.
6. Lubricate the dry granules with 0.5% magnesium stearate.
7. The lubricated granules are compressed on a suitable tablet press.

## Intravenous Injection

| Ingredients | | Per ml. |
|---|---|---|
| 1. Active ingredient | | 1.0 mg. |
| 2. pH 4.0 Buffer solution | q.s. to | 1.0 ml. |

Procedure:

1. Dissolve the active ingredient in the buffer solution.
2. Aseptically filter the solution from Step #1.
3. The sterile solution is now aseptically filled into sterile ampoules.
4. The ampoules are sealed under aseptic conditions.

## Intramuscular Injection

| Ingredients | | Per ml. |
|---|---|---|
| 1. Active ingredients | | 5.0 mg. |
| 2. Isotonic Buffer solution 4.0 | q.s. to | 1.0 ml. |

Procedure:

1. Dissolve the active ingredient in the buffer solution.
2. Aseptically filter the solution from Step #1.
3. The sterile solution is now aseptically filled into sterile ampoules.
4. The ampuls are sealed under aseptic conditions.

Suppositories

| | Ingredients | Per Supp. |
|---|---|---|
| 1. | Active ingredient | 10.0 mg. |
| 2. | Polyethylene Glycol 1000 | 1350.0 mg. |
| 3. | Polyethylene Glycol 4000 | 450.0 mg. |

Procedure:

1. Melt 2 and 3 together and stir until uniform.
2. Dissolve #1 in the molten mass from Step #1 and stir until uniform.
3. Pour the molten mass from Step #2 into suppository molds and chill.
4. Remove the suppositories from molds and wrap.

Therapeutic compositions having cardiac arrhythmia inhibiting activity in dosage unit form, comprising a pharmaceutical carrier and a cardiac arrhythmic inhibiting amount of a compound of Formula I or a pharmaceutically acceptable acid addition salt thereof are therefore an embodiment of this invention.

Various modifications and equivalents will be apparent to one skilled in the art and may be made in the compounds, method, and compositions of the present invention without departing from the spirit or scope thereof, and it is therefore to be understood that the invention is to be limited only by the scope of the appended claims.

CLAIMS FOR THE DESIGNATED STATES: BE, FR, DE, IT, LU, NL, SE, CH, LI and GB

1. A compound of general formula I:

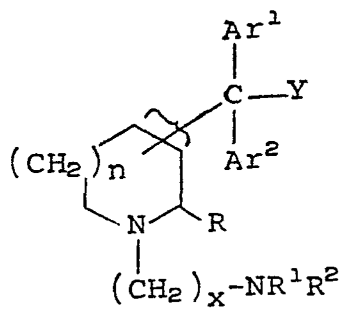

(I)

wherein:

n is zero, one or two;

$Ar^1$ and $Ar^2$, which may be the same or different, are selected from 2-, 3- and 4-pyrido, phenyl and phenyl substituted by 1 to 3 radicals, which may be the same or different and selected from loweralkyl, loweralkoxy, halogen and trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl, loweralkoxy and phenyl-loweralkyl wherein phenyl may be substituted by halogen, loweralkyl or loweralkoxy, and $R^1$ and $R^2$ may be the same or different, or, taken together with the adjacent nitrogen atom, may form a heterocyclic residue selected from pyrrolidino, piperidino, 4-phenylpiperidino, 2,6-diloweralkyl-piperidino, 4-hydroxy-4-phenylpiperidino, 4-cyano-4-phenylpiperidino, piperazino, 4-loweralkylpiperazino, 4-phenylpiperazino, (4-phenyl-loweralkyl)-piperazino

and 4-morpholino radicals; or a pharmaceutically acceptable acid addition salt thereof.

2. A compound as claimed in Claim 1, wherein each of $Ar^1$ and $Ar^2$ represents a phenyl group.

3. A compound as claimed in Claim 1 or 2, wherein x is 2 or 3.

4. A compound as claimed in Claim 1, 2 or 3, wherein $R^1$ and $R^2$ both represent loweralkyl or, together with the adjacent nitrogen atom, form a pyrrolidino residue.

5. 1-[2-(Dimethylamino)ethyl]-α,α-diphenyl-3-pyrrolidineacetonitrile,
1-[2-(dimethylamino)ethyl]-α,α-diphenyl-3-pyrrolidine-acetamide,
1-[3-(dimethylamino)propyl]-α,α-diphenyl-3-pyrrolidineacetamide,
1-[2-(diethylamino)ethyl]-α,α-diphenyl-3-pyrrolidine-acetamide,
α,α-diphenyl-1-[2-(1-pyrrolidinyl)ethyl]-3-pyrrolidineacetamide,
1-[2-(dimethylamino)ethyl]-α,α-diphenyl-3-piperidine-acetamide or
1-[2-(dimethylamino)ethyl]-α,α-diphenyl-4-piperidine-acetamide or a pharmaceutically acceptable acid addition salt thereof.

6. A compound having the formula

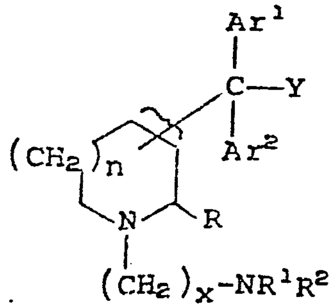

(I)

wherein:

n is zero, one or two;

$Ar^1$ and $Ar^2$, which may be the same or different, are selected from 2-, 3- and 4-pyrido, phenyl and phenyl substituted by 1 to 3 radicals which may be the same or different and selected from loweralkyl, loweralkoxy, halogen and trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl, loweralkoxy and phenyl-loweralkyl, wherein phenyl may be substituted by halogen, loweralkyl, or loweralkoxy, and $R^1$ and $R^2$ may be the same or different, or, taken together with the adjacent nitrogen atom, may form a heterocyclic residue selected from pyrrolidino, piperidino, 4-phenylpiperidino, 2,6-diloweralkyl-piperidino, 4-hydroxy-4-phenylpiperidino, 4-cyano-4-phenylpiperidino, piperazino, (4-loweralkyl)piperazino and 4-morpholino radicals; or a pharmaceutically acceptable acid addition salt thereof, for use in human or veterinary medicine.

7. A compound as claimed in any one of Claims 2 to 6 for use in human or veterinary medicine.

8. A therapeutic composition for the treatment of cardiac arrhythmias comprising a) an effective amount of a compound selected from the group having the formula:

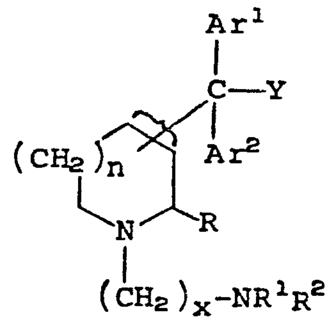

wherein:

n is zero, one or two;

$Ar^1$ and $Ar^2$, which may be the same or different, are selected from 2-, 3- and 4-pyrido, phenyl and phenyl substituted by 1 to 3 radicals which may be the same or different and selected from loweralkyl, loweralkoxy, halogen and trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl, loweralkoxy and phenyl-loweralkyl wherein phenyl may be substituted by halogen, loweralkyl or loweralkoxy, and $R^1$ and $R^2$ may be the same or different, or, taken together with the adjacent nitrogen atom, may form a heterocyclic residue selected from pyrrolidino, piperidino, 4-phenylpiperidino, 2,6-diloweralkylpiperidino, 4-hydroxy-4-phenylpiperidino, 4-cyano-4-

phenylpiperazino, (4-phenyl-loweralkyl)-piperazino or 4-morpholino radicals, or a pharmaceutically acceptable salt thereof, and

(b) a pharmaceutically acceptable carrier therefor.

9. A therapeutic composition for the treatment of cardiac arrhythmias comprising a) an effective amount of a compound as claimed in any one of Claims 2 to 5 and b) a pharmaceutically acceptable carrier therefor.

10. The use of a compound of general formula I

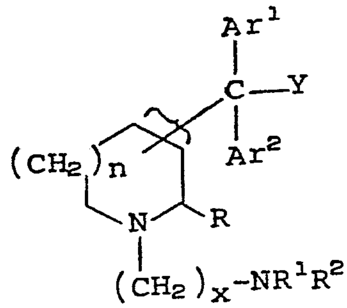

(I)

wherein:

n is zero, one or two;

$Ar^1$ and $Ar^2$, which may be the same or different, are selected from 2-, 3- and 4-pyrido, phenyl and phenyl substituted by 1 to 3 radicals, which may be the same or different and selected from loweralkyl, loweralkoxy, halogen and trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl, loweralkoxy and phenyl-loweralkyl wherein phenyl may be

substituted by halogen, loweralkyl or loweralkoxy, and $R^1$ and $R^2$ may be the same or different, or, taken together with the adjacent nitrogen atom, may form a heterocyclic residue selected from pyrrolidino, piperidino, 4-phenylpiperidino, 2,6-diloweralkylpiperidino, 4-hydroxy-4-phenylpiperidino, 4-cyano-4-phenylpiperidino, piperazino, 4-loweralkylpiperazino, 4-phenylpiperazino, (4-phenyl-loweralkyl)-piperazino and 4-morpholino radicals; or a pharmaceutically acceptable acid addition salt thereof, in the manufacture of a cardiac antiarrhythmic agent.

11. The use of a compound as claimed in any one of Claims 1 to 5 in the manufacture of a cardiac antiarrhythmic agent.

0178946

CLAIMS FOR THE DESIGNATED STATE: AT

1. A process for the preparation of a compound of general formula I

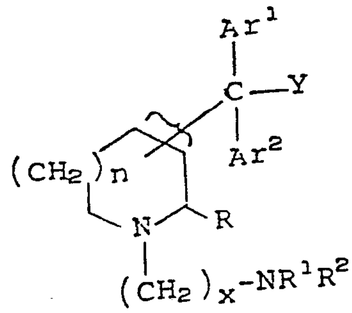

(I)

wherein:

n is zero, one or two;

$Ar^1$ and $Ar^2$, which may be the same or different, are selected from 2-, 3- and 4-pyrido, phenyl and phenyl substituted by 1 to 3 radicals, which may be the same or different and selected from loweralkyl, loweralkoxy, halogen and trifluoromethyl;

Y represents aminocarbonyl or cyano;

x is from two to five;

R represents hydrogen or loweralkyl;

$R^1$ and $R^2$ are selected from hydrogen, loweralkyl, phenyl, phenyl substituted by halogen, loweralkyl, loweralkoxy and phenyl-loweralkyl wherein phenyl may be substituted by halogen, loweralkyl or loweralkoxy, and $R^1$ and $R^2$ may be the same or different, or, taken together with the adjacent nitrogen atom, may form a heterocyclic residue selected from pyrrolidino, piperidino, 4-phenylpiperidino, 2,6-diloweralkyl-piperidino, 4-hydroxy-4-phenylpiperidino, 4-cyano-4-phenylpiperidino, piperazino, 4-loweralkyl-piperazino, 4-phenylpiperazino, (4-phenyl-loweralkyl)-piperazino and 4-morpholino radicals; or a pharmaceutically acceptable acid addition salt thereof, the process comprising

(A) either reacting a compound of general formula II

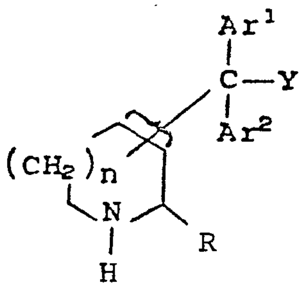

(II)

wherein n, $Ar^1$, $Ar^2$, Y and R are as defined for general formula I above, with a compound of general formula (III)

$$halo-(CH_2)_x-NR^{1A}R^{2A} \qquad (III)$$

wherein halo represents a halogen atom, x is as defined in formula I above and $R^{1A}$ and $R^{2A}$ are as defined for $R^1$ and $R^2$ respectively in formula I, provided that $R^{1A}$ and/or $R^{2A}$ cannot be hydrogen or unprotected piperazine, but can additionally represent a halogen atom or, together with the adjacent nitrogen atom form 1-phthalimido, 4 substituted piperazine or

O
‖
-N-C-O-loweralkyl , and subsequently, when either or
|
loweralkyl

both of $R^{1A}$ and $R^{2A}$ represent a substituent not within the definition of either or both of $R^1$ and $R^2$, converting a substituent defined by $R^{1A}$ to a substituent defined by $R^1$ and/or converting a substituent defined by $R^{2A}$ to a substituent defined by $R^2$, to obtain a compound as defined in general

formula I ;

(B) or reacting a compound of general formula IV

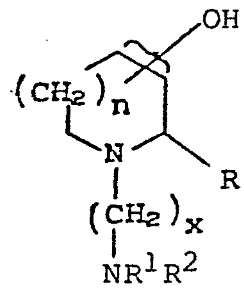

(IV)

wherein R, $R^1$, $R^2$, x and n are as defined for general formula I above,

first with a compound of general formula V

halo-$SO_2W$ (V)

wherein halo represents a halogen atom and W represents a loweralkyl or phenyl group, the phenyl group optionally being substituted by one or more non-interfering radicals, and then with a compound of general formula VI

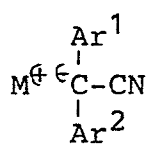

(VI)

wherein $M^+$ represents an alkali metal ion and $Ar^1$ and $Ar^2$ are as defined for general formula I above, to obtain a compound as defined in general formula I, except that -Y represents -CN;

(C) and in either case optionally converting a compound of general formula I so formed into another compound of formula I and/or into a salt thereof.

2. A process as claimed in Claim 1, wherein each of $Ar^1$ and $Ar^2$ represents a phenyl group.

3. A process as claimed in Claim 1 or 2, wherein x is 2 or 3.

4. A process as claimed in Claim 1, 2 or 3, wherein $R^1$ and $R^2$ both represent loweralkyl or, together with the adjacent nitrogen atom, form a pyrrolidino residue.

5. A process for the preparation of 1-[2-(dimethylamino)ethyl]-α,α-diphenyl-3-pyrrolidineacetonitrile, or a salt thereof, which comprises either reacting α,α-diphenyl-3-pyrrolidineacetonitrile with 2-(dimethyl-(amino)ethyl chloride or reacting 1-[2-(dimethylamino)ethyl]-3-pyrrolidinol first with methylsulphonyl chloride and then with diphenylacetonitrile, and in either case optionally forming a salt thereof.

6. A process for the preparation of 1-[2-(dimethylamino)ethyl]-α,α-diphenyl-3-pyrrolidineacetamide, or a salt thereof, which comprises either reacting α,α-diphenyl-3-pyrrolidinylacetamide with 2-(dimethylamino)ethyl chloride or reacting 1-[2-(dimethylamino)-ethyl]-3-pyrrolidinol first with methylsulphonyl chloride and then with diphenylacetonitrile and subsequently hydrolyzing the product to the corresponding acetamide, and in either case optionally forming a salt thereof.

7. A process for the preparation of 1-[2-(dimethylamino)propyl]-α,α-diphenyl-3-pyrrolidineacetamide, or a salt thereof, which comprises either reacting α,α-

diphenyl-3-pyrrolidinylacetamide with 2-(dimethyl-amino)propyl chloride or reacting 1-[2-(dimethylamino)-propyl]-3-pyrrolidinol first with methylsulphonyl chloride and then with diphenylacetonitrile and subsequently hydrolysing the product to the corresponding acetamide, and in either case optionally forming a salt thereof.

8. A process for the preparation of 1-[2-(diethyl-amino)ethyl]-α,α-diphenyl-3-pyrrolidineacetamide, or a salt thereof, which comprises either reacting α,α-diphenyl-3-pyrrolidinylacetamide with 2-(diethyl-amino)ethyl chloride or reacting 1-[2-(diethylamino)-propyl]-3-pyrrolidinol first with methylsulphonyl chloride and then with diphenylacetonitrile and subsequently hydrolysing the product to the corresponding acetamide, and in either case optionally forming a salt thereof.

9. A process for the preparation of 1-[2-(1-pyrrolidinyl)ethyl]-α,α-diphenyl-3-pyrrolidineacet-amide, or a salt thereof, which comprises either reacting α,α-diphenyl-3-pyrrolidinylacetamide with 2-(1-pyrrolidinyl)ethyl chloride or reacting 1-[2-(1-pyrrolidinyl)ethyl]-3-pyrrolidinol first with methylsulphonyl chloride and then with diphenylacetonitrile and subsequently hydrolysing the product to the corresponding acetamide, and in either case optionally forming a salt thereof.

10. A process for the preparation of 1-[2-(dimethyl-amino)ethyl]-α,α-diphenyl-3-piperidineacetamide, or a salt thereof, which comprises either reacting α,α-

diphenyl-3-piperidinylacetamide with 2-(dimethyl-amino)ethyl chloride or reacting 1-[2-(dimethylamino)-ethyl]-3-piperidinol first with methylsulphonyl chloride and then with diphenylacetonitrile and subsequently hydrolysing the product to the corresponding acetamide, and in either case optionally forming a salt thereof.

11. A process for the preparation of 1-[2-(dimethyl-amino)ethyl]-α,α-diphenyl-4-piperidineacetamide, or a salt thereof, which comprises either reacting α,α-diphenyl-4-piperidinylacetamide with 2-(dimethyl-amino)ethyl chloride or reacting 1-[2-(dimethylamino)-ethyl]-4-piperidinol first with methylsulphonyl chloride and then with diphenylacetonitrile and subsequently hydrolysing the product to the corresponding acetamide, and in either case optionally forming a salt thereof.